# EUROPEAN PATENT SPECIFICATION

(11) **EP 2 451 830 B1**
(45) Date of publication and mention of the grant of the patent: **30.03.2016**
(21) Application number: 10734839.3
(22) Date of filing: 07.07.2010
(51) Int. Cl.: C07K 14/415, C12N 15/82

(54) **METHOD FOR INCREASING THE RESISTANCE OF A PLANT OR A PART THEREOF TO A PATHOGEN, METHOD FOR SCREENING THE RESISTANCE OF A PLANT OR PART THEREOF TO A PATHOGEN, AND USE THEREOF**
VERFAHREN ZUR ERHÖHUNG DER RESISTENZ EINER PFLANZE ODER EINES TEILES DAVON GEGEN EIN PATHOGEN, VERFAHREN ZUM SCREENING DER RESISTENZ ODER EINES TEILES DAVON GEGEN EIN PATHOGEN UND VERWENDUNG DAVON
PROCÉDÉ POUR AUGMENTER LA RÉSISTANCE D'UNE PLANTE OU UNE PIÈCE ASSOCIÉE À UN PATHOGÈNE, PROCÉDÉ DE CRIBLAGE DE LA RÉSISTANCE D'UNE PLANTE OU PIÈCE ASSOCIÉE À UN PATHOGÈNE, ET UTILISATION ASSOCIÉE

(30) Priority: 08.07.2009 EP 09164957
(43) Date of publication of application: 16.05.2012
(73) Proprietor: Wageningen Universiteit, 6708 PB Wageningen (NL)
(72) Inventor: DE WIT, Peter Jozef Gerard Marie, NL-3911 XM Rhenen (NL); STERGIOPOULOS, Ioannis, NL-6709 PD Wageningen (NL); KEMA, Gerrit Haatje Jan, NL-3904 HM Veenendaal (NL)
(74) Representative: V.O.
(86) International application number: PCT/NL2010/050433
(87) International publication number: WO 2011/005090

(56) References cited:
- WO-A1-96/35790
- TAKKEN F L W ET AL: "A FUNCTIONAL CLONING STRATEGY, BASED ON A BINARY PVX-EXPRESSION VECTOR, TO ISOLATE HR-INDUCING CDNAS OF PLANT PATHOGENS" PLANT JOURNAL, BLACKWELL SCIENTIFIC PUBLICATIONS, OXFORD, GB, vol. 24, no. 2, 1 January 2000 (2000-01-01), pages 275-283, XP001042390 ISSN: 0960-7412
- VAN DER HOORN RENIER A L ET AL: "Agroinfiltration is a versatile tool that facilitates comparative analyses of Avr9/Cf-9-induced and Avr4/Cf-4-induced necrosis" MOLECULAR PLANT-MICROBE INTERACTIONS, vol. 13, no. 4, April 2000 (2000-04), pages 439-446, XP002555466 ISSN: 0894-0282
- XIAO WENKAI ET AL: "Genome-wide isolation of resistance gene analogs in maize (Zea mays L.)" THEORETICAL AND APPLIED GENETICS ; INTERNATIONAL JOURNAL OF PLANT BREEDING RESEARCH, SPRINGER, BERLIN, DE, vol. 113, no. 1, 11 April 2006 (2006-04-11), pages 63-72, XP019417687 ISSN: 1432-2242
- STERGIOPOULOS IOANNIS ET AL: "Tomato Cf resistance proteins mediate recognition of cognate homologous effectors from fungi pathogenic on dicots and monocots" PROCEEDINGS OF THE NATIONAL ACADEMY OF SCIENCES OF THE UNITED STATES OF AMERICA, vol. 107, no. 16, April 2010 (2010-04), pages 7610-7615, XP002599195 ISSN: 0027-8424

## Description

### INTRODUCTION

The present invention relates to the field of plant biotechnology. More in particular, the present invention relates to methods for increasing the resistance of a plant or part thereof that is susceptible to infection with a pathogen comprising an ortholog of the Avr4 protein of *Cladosporium fulvum,* wherein said plant is not a tomato plant. The invention also relates to methods for screening the resistance of a plant or a part thereof to at least one pathogen, wherein said plant is not a tomato plant. The invention further relates to the use of such methods.

### BACKGROUND OF THE INVENTION

The world's most important agricultural plants are still highly susceptible to pathogen-induced diseases that can severely affect their growth and reproductive rate. In some cases, even the very existence of such plants may be threatened. For example, the leading banana cultivar "Gros Michel" was discovered in the 1820s and was grown and consumed throughout the world until the 1920s. It was almost completely eradicated in only a few decades by the Panama Disease, caused by the soilborne hyphomycete fungus, *Fusarium oxysporum* f. sp. *cubense.* It has been hypothesized that its successor, the cultivar "Cavendish", may become unviable for large-scale cultivation in the next 10-20 years due to a similar disease. Evenly grim prospects have also been foresighted for other agricultural plants such as potato, corn, soybean and wheat.

Thus, as the production values of agricultural plants are very important for keeping up with the world's ever increasing food demand, there is a dire need to improve their resistance to pathogens. Of these plants, banana and sugar beet plants share their susceptibility to fungal pathogens, belonging to the class of Dothideomycetes.

The production value of banana plants (*Musa* ssp., including plantains and other types of cooking bananas) is only succeeded by that of maize, rice and wheat. Annually, nearly 100 million tons of bananas are produced in about 120 countries worldwide. As mentioned above, bananas are very prone to fungal infections. Of these infections, Panama Disease and, more recently, the Sigatoka Disease Complex belong to the most devastating ones. The Sigatoka Disease Complex involves three phylogenetically closely related species of the Dothideomycete family of *Mycosphaerellaceae,* including *M. musicola, M. fijiensis* and the recently characterized *M. eumusae* (reviewed in Jones 2000, 2003, Arzanlou *et al.* 2007). The disease is a serious threat to banana plantations around the world, causing extensive economic losses. The three species emerged on bananas during the last century with *M. musicola* (causing yellow Sigatoka) appearing first in South East Asia in 1902 and gradually being replaced by *M. fijiensis* (causing black Sigatoka or black leaf streak) that appeared in the Sigatoka valley of the Fiji islands during the 1960s. The third species, *M. eumusae,* causes Eumusae leaf spot and was identified as a new constituent of the Sigatoka complex on bananas during the 1990s. Currently, the black Sigatoka is the most destructive disease of banana worldwide, reducing yields by more than 38% (Marin *et al.* 1991).

Sugar beet (*Beta vulgaris L*.) is a member of the Chenopodiaceae family, and accounts for 30% of the world's sugar production. Like wheat and bananas, it is also prone to infection by fungi that belong to the class of Dothideomycetes. The species *Cercospora beticola* is particularly well known for its capability to infect sugar beets. Sugar beets that have been infected by *C. beticola* can easily be recognized by distinctive round leaf spots that are often surrounded by a red ring.

Another type of plant that is commercially grown on a very large scale and that is prone to viral and fungal infection is the tobacco plant (genus *Nicotiana*). World tobacco production is projected to reach over 7.1 million tonnes of tobacco leaf in the year 2010, up from 5.9 million tonnes in 1997/99. About 100 countries produce tobacco. The major producers are China, India, Brazil, the US, Turkey, Zimbabwe and Malawi, which together provide over 80 percent of the world's tobacco production. Of these producers, China alone accounts for over 35 percent of world production. Tobacco plants are also susceptible to fungal infestation by Dothideomycetes. For example, infection of tobacco leaves with *Cercospora nicotianae* is a main cause of wide-spread tobacco plant diseases such as barn rot and frogeye leaf spot.

Currently, methods for controlling these diseases in commercial plantations are mostly restricted to extensive and repeated application of fungicides. In the past, the annual worldwide costs for these applications were already estimated at $ 2.5 billion for bananas alone, at that time accounting for 27% of the banana production costs (Stover 1980, Stover and Simmonds 1987). It may be clear that such extensive application is not only expensive, but in addition it poses serious threats to human and animal health and to the environment. Furthermore, it has been demonstrated that the intensive application of fungicides has already resulted in the development of resistant fungal strains (Marin *et al.* 2003).

Thus, alternative methods for increasing the resistance of plants to their pathogens are needed. Preferably, such methods avoid the application of chemical compounds such as fungicides.

Such methods are known in the art, as for example methods to genetically transform banana (Crouch *et al.* 1998, Report 1997, Sági 1997). These methods have however not proven successful yet. Thus, the need for improving the resistance of agricultural plants to their pathogens remains.

Dothideomycetes are a class of Ascomycete fungi that comprises about 50 families, 650 genera and 6300 known species. Traditionally most of its members were included in the clade Loculoascomycetes. The class contains several important plant pathogens, such as *Stagonospora nodorum* (causing *Stagonospora* leaf blotch of cereals), *Venturia inaequalis* (causing leaf scabs of many plants including apple and pear) and *Ascochyta* species (causing leaf spot of legumes including pea, chickpea, bean and many others).

*Cladosporium fulvum* is a biotrophic Dothidiomycete that causes leaf mould of tomato (*Lycopersicon esculentum*). *C. fulvum* secretes effector proteins into the apoplast of the infected plants to facilitate disease development (Thomma *et al.* 2005). These effector proteins can be recognized by cognate *C. fulvum* (Cf) resistance proteins present in resistant tomato lines. Recognition by Cf proteins results in a rapid elicitation of plant defense responses, culminating in the hypersensitive response (HR), a type of programmed cell death. Cells surrounding the initial infection site quickly die and in this way, further growth of the biotrophic fungus is prevented (De Wit *et al.* 2008; Joosten & De Wit 1999; Thomma *et al.* 2005).

To date, four avirulence (*Avr*) genes have been cloned from *C*. *fulvum* and they all encode small cysteine-rich proteins that are secreted during infection. These are the Avr2, Avr4, Avr4E, and Avr9 effector proteins, whose recognition in tomato is mediated by the cognate Cf resistance proteins Cf2, Cf4, Cf4E, and Cf9, respectively (De Wit *et al.* 2008; Joosten & De Wit 1999; Thomma *et al.* 2005). Four additional extracellular proteins (Ecps), namely Ecp1, Ecp2, Ecp4, and Ecp5 have been characterized from *C. fulvum* that invoke an HR in tomato lines that carry a cognate matching *Cf-Ecp* gene (De Kock *et al.* 2005; Laugé *et al.* 2000). Recently, also the Ecp6 and Ecp7 effectors have been identified but so far no tomato lines have been reported that carry matching cognate *Cf-Ecp* genes mediating their recognition (Bolton *et al.* 2008).

Although demonstrated for only a few, all Avrs and Ecps are assumed to be virulence factors (Bolton *et al.* 2008; Thomma *et al.* 2005; van Esse *et al.* 2007; van Esse *et al.* 2008) The Avr2 effector inhibits extracellular plant cysteine proteases, including Rcr3, Pip1, aleurain and TDI6 (Krüger *et al.* 2002; Rooney *et al.* 2005; Shabab *et al.* 2008). *C. fulvum* strains producing Avr2 trigger HR in tomato lines that carry *Rcr3* and the cognate *Cf-2* resistance gene (Dixon *et al.* 1996; Luderer *et al.* 2002b). The Avr4 effector is a chitin-binding protein that protects the fungal cell wall against the deleterious effects of plant chitinases (van den Burg *et al.* 2006; van den Burg *et al.* 2004; van den Burg *et al.* 2003).

Tomato plants that carry the cognate *Cf-4* resistance gene recognize Avr4-producing strains of *C. fulvum* and initiate an HR (Joosten *et al.* 1994; Joosten *et al.* 1997, Westerink *et al.* 2004). The nucleic acid sequence of the tomato *Cf-4* gene is disclosed in WO 96/35790, along with the encoded amino acid sequence of Cf4. WO 96/35790 also describes a method for increasing the resistance in a plant to *C. fulvum* by transforming the plant with a nucleic acid sequence encoding for the *Cf-4* gene, thereby providing the plant with Cf4 proteins that recognize the Avr4 effector protein. However, as the method confers a plant with resistance to *C. fulvum* strains that produce Avr4, its application is limited to plants that are susceptible to infection with *C. fulvum,* such as a tomato plant and tobacco. The method is not suitable for increasing resistance in plants that are susceptible to pathogens other than *C. fulvum,* i.e. plants such as banana, sugar beet, tobacco, maize, and celery. A method for increasing the resistance to pathogens of plants other than tomato plants is thus still direly needed.

### SUMMARY OF THE INVENTION

The current invention provides such a method. Provided is a method for increasing the resistance of a plant or part thereof that is susceptible to infection with a pathogen comprising an ortholog of the Avr4 protein of *Cladosporium fulvum,* wherein said method comprises transforming said plant or part thereof with a nucleic acid encoding for Cf4 or a functional homologue thereof and wherein said plant is not a tomato or tobacco plant. Preferably, said plant or part thereof is selected from the group consisting of a banana plant, a wheat plant, a sugar beet plant, a maize plant, and a celery plant. Preferably, said functional homologue is an Hcr9-Avr4 protein.

In one embodiment, said transforming comprises introducing a nucleic acid delivery vehicle. Preferably, said delivery vehicle is an expression vector. More preferably, said expression vector is a virus-based expression vector. Preferably, said virus is an adenovirus or a retrovirus.

Also provided is a method for screening the resistance of a plant or a part thereof to at least one pathogen with the proviso that said pathogen is not *Cladosporium fulvum,* said method comprising: a) screening said at least one pathogen for presence of the Avr4 protein of *Cladosporium fulvum* or an ortholog thereof, b) selecting at least one pathogen comprising said Avr4 protein of *Cladosporium fulvum* or an ortholog thereof, c) introducing said at least one pathogen to said plant or part thereof, and d) screening said plant or part thereof for the absence or presence of a defense response to said at least one pathogen, wherein said plant is not a tomato or tobacco plant. Preferably, said plant or part thereof is selected from the group consisting of a banana plant, a wheat plant, a sugar beet plant, a maize plant and a celery plant. Preferably, said ortholog comprises a chitin-binding domain similar to that found in members of the CBM14 superfamily of chitin-binding proteins. More preferably, said ortholog comprises an amino acid sequence that is selected from the group consisting of SEQ ID NO: 1, SEQ ID NO: 2, SEQ ID NO: 3, SEQ ID NO: 4, SEQ ID NO: 5 and SEQ ID NO: 6., more preferably selected from the group consisting of SEQ ID NO: 2, SEQ ID NO: 3, SEQ ID NO: 4, SEQ ID NO: 5 and SEQ ID NO: 6.

Preferably, the pathogen in the methods according to the invention is a fungus, with the proviso that said fungus is not *Cladosporium fulvum.* More preferably, said fungus is a Dothideomycete. Even more preferably, said Dothideomycete is selected from the group consisting of *Mycosphaerella fijiensis, Cercospora nicotianae, Cercospora beticola, Cercospora zeina*, and *Cercospora apii.*

Further provided is the use of the methods according to the invention for increasing the resistance of a plant or part thereof to at least one pathogen, wherein said plant is not a tomato or tobacco plant. Even further provided is the use of the methods according to the invention for screening the resistance of a plant or a part thereof to at least one pathogen, wherein said plant is not a tomato or tobacco plant. Preferably, said plant or part thereof is selected from the group consisting of a banana plant, a wheat plant, a sugar beet plant, a maize plant and a celery plant. Preferably, said pathogen is a fungus, with the proviso that said fungus is not *Cladosporium fulvum.* More preferably, said fungus is a Dothideomycete. Even more preferably, said Dothideomycete is selected from the group consisting of *Mycosphaerella fijiensis, Cercospora nicotianae, Cercospora beticola, Cercospora zeina,* and *Cercospora apii.*

It is further submitted that the present invention does not comprise any of the methods or plants that are disclosed in WO 96/35790. This specifically relates to a method for introducing Cf4 in plants to provide resistance to *C. fulvum,* or plants transgenic for the Cf4 sequence. Also excluded from the present invention are plants that are transgenic for both the Cf4 and the Avr4 sequences. Further excluded are tomato and tobacco plants transgenic for the Cf4 sequence or the Avr4 sequence.

### LEGENDS TO THE FIGURES

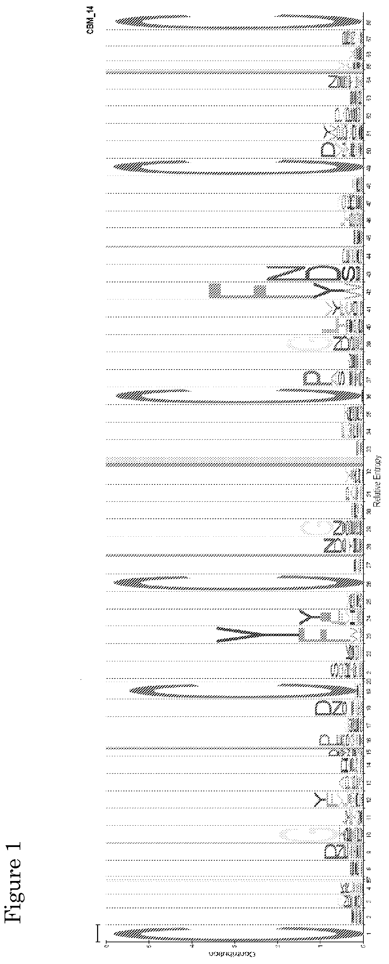

Figure 1: Conservation of the chitin-binding domain amongst CBM_14 proteins based on Hidden Markov Models (HMM). The cysteine residues (denoted as large C) are always conserved.

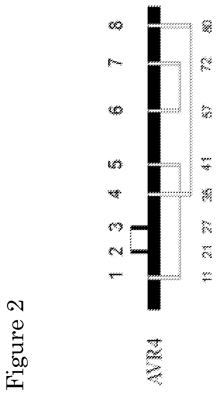

Figure 2: The disulfide bond pattern of the *C. fulvum* Avr4 (van den Burg *et al.* 2003). Connected open bars represent three conserved disulfide bonds of the invertebrate chitin-binding domain (inv ChBD). Connected filled bands represent additional bonds. A similar disulfide bond pattern is expected for the *M. fijiensis* and *Cercopsora* Avr4-orthologs as well.

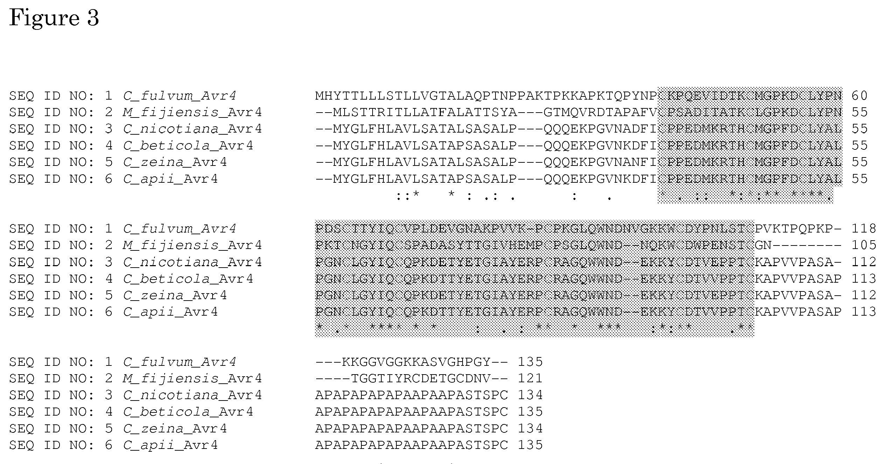

Figure 3: Alignment of the amino acid sequences of the Avr4-protein from *C. fulvum* (SEQ ID NO: 1) and the orthologs thereof from *M. fijiensis* (SEQ ID NO: 2), *C. nicotianae* (SEQ ID NO: 3), *C. beticola* (SEQ ID NO: 4), *C. zeina* (SEQ ID NO: 5), and *C. apii* (SEQ ID NO: 6). A predicted chitin-binding domain (highlighted) is present in all three sequences. Conserved cysteine residues (in bold) that are involved in the formation of disulfide-bridges and are important for protein stability are also indicated.

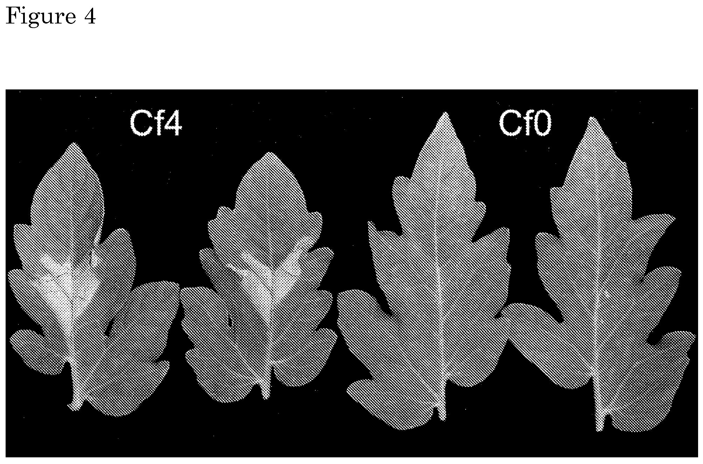

Figure 4: Infiltrations of the *M. fijiensis* Avr4 in *Cf4* (left) and *Cf0* (right) tomato plants. For the purpose of this experiment, the *Pichia pastoris* expression system was used to produce MfAvr4. In this picture crude MfAvr4 extracts isolated from culture filtrates of *P. pastoris* producing MfAvr4 were infiltrated in Cf-4 (containing the matching cognate Cf4 resistance protein) and Cf-0 (no Cf4 resistance protein present) tomato plants. Infiltrations with MfAvr4 clearly result in an HR in the presence of the *Cf-4* resistance gene. Pictures were taken 7 days post injection.

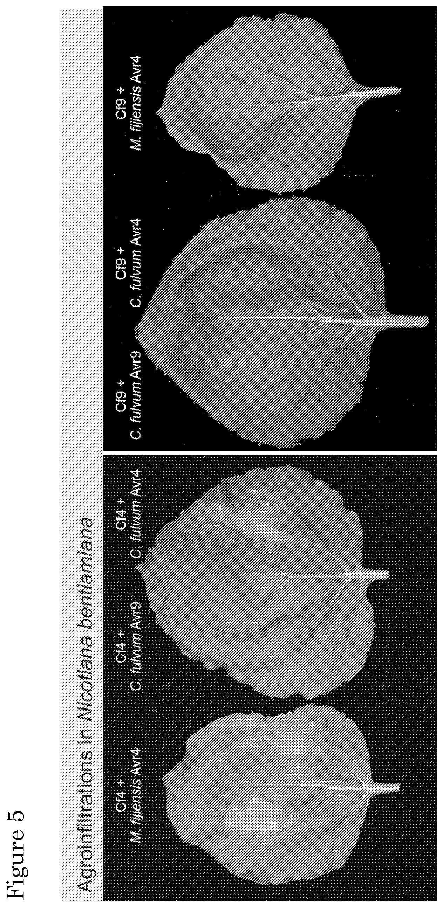

Figure 5: Transient co-expression of *Cf genes* from tomato and *Avr* genes from *C. fulvum* and *M. fijiensis* in *Nicotiana benthamiana* by agroinfiltration. Four transient expression *Agrobacterium* cultures were mixed in a 1:1 ratio and infiltrated. Pictures were taken six days post infiltration. The pictures demonstrate that co-expression of the *M. fijiensis Avr4 (MfAvr4)* and the tomato *Cf-4* gene results in HR on *N. benthamiana* leaves (left panel). However, HR is not induced when *MfAvr4* is co-expressed with *Cf-9,* indicating the specificity of the interaction between Cf4 and MfAvr4 (right panel). As a control for this experiment, the *C. fulvum Avr4* (Avr4) and *Avr9* were co-expressed with *Cf-4* and *Cf-9.* In this case, HR is only observed upon co-expression of *Avr4* and *Cf-4* (left panel) or *Avr9* with *Cf-9* (right panel), as expected.

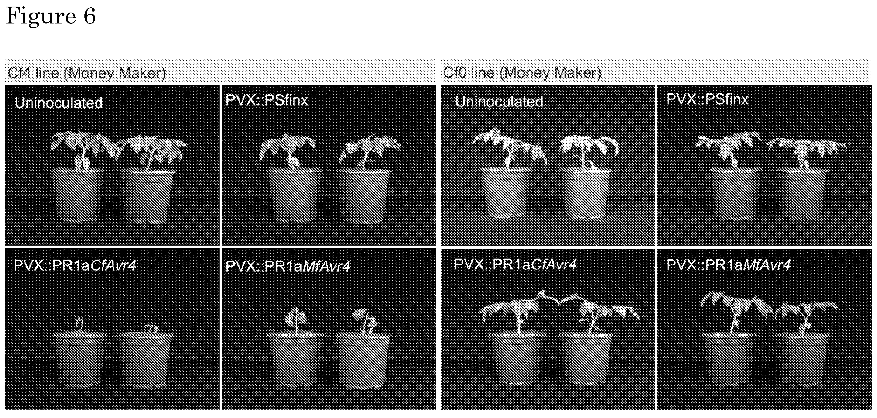

Figure 6: Specific HR induced responses in a MM-Cf4 tomato line after transient expression of the *MfAvr4,* using a PVX-based expression system. Cotyledons from a four-week-old tomato line carrying the *Cf-4* resistance gene (MM-Cf4) have been inoculated with A. *tumefaciens* transformants of PVX::PR1a*MfAvr4*. These transformants harbor the *M. fijiensis Avr4* cDNA in the binary PVX-based vector pSfinx, downstream of the 35S promoter and fused to the PR1a signal sequence from tobacco. HR occurs only in leaflets of the MM-Cf4 tomato line but not in leaflets of the MM-Cf0 line, thus showing the specific recognition of the MfAvr4 by Cf4. As controls PVX containing the empty pSfinx vector (PVX::pSfinx) and the *C. fulvum Avr4* in a binary vector (PVX::PR1a*CfAvr4*), were used. As expected, the PVX-based expression of the *C. fulvum Avr4* results in an HR only in the MM-Cf4 line but not in the MM-Cf0 line. Only mosaic symptoms were observed upon inoculations of the MM-Cf4 and Cf-0 lines with PVX containing the empty pSfinx vector. Pictures were taken 14 days after inoculation.

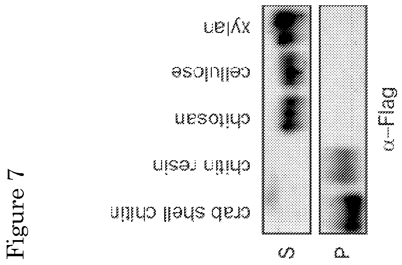

Figure 7: Specific binding of MfAvr4 to chitin but not to other polysaccharides. *Pichia pastoris* culture filtrate containing MfAvr4 was incubated together with the insoluble polysaccharides crab shell chitin, chitin-agarose resin chitosan, cellulose, and xylan at ambient temperature for 2 hrs. Chitin-binding affinity was determined by loading both bound (B) and unbound protein (S) protein fraction on separate SDS-PAGE gels. His-Flag-tagged MfAvr4 was detected using an anti-Flag tag monoclonal antibody. Equal amounts of the bound and unbound protein were loaded on the gel. To this aim, the protein fraction in the supernatant was precipitated in the presence of 10% TCA (w/v). S, protein that remained in the concentrated supernatant fraction; P, protein bound to insoluble polysaccharide fraction. MfAvr4 is specifically precipitated with chitin and specific binding was confirmed in five independent replicate experiments.

### DETAILED DESCRIPTION OF THE INVENTION

### Definitions

As used herein, the term "plant or part thereof" means any complete or partial plant, including single cells and cell tissues such as plant cells that are intact in plants, cell clumps and tissue cultures from which plants can be regenerated. Examples of plant parts include, but are not limited to, single cells and tissues from pollen, ovules, leaves, embryos, roots, root tips, anthers, flowers, fruits, stems shoots, and seeds; as well as pollen, ovules, leaves, embryos, roots, root tips, anthers, flowers, fruits, stems, shoots, scions, rootstocks, seeds, protoplasts, calli, and the like.

As used herein, the term "variety" is as defined in the UPOV treaty and refers to a group of similar plants that by structural or genetic features and/or performance can be distinguished from other varieties within the same species.

The term "cultivar" (for cultivated variety) as used herein is defined as a variety that is not normally found in nature but that has been cultivated by humans, i.e. having a biological status other than a "wild" status, which "wild" status indicates the original non-cultivated, or natural state of a plant or accession. The term "cultivar" includes, but is not limited to, semi-natural, semi-wild, weedy, traditional cultivar, landrace, breeding material, research material, breeder's line, synthetic population, hybrid, founder stock/base population, inbred line (parent of hybrid cultivar), segregating population, mutant/genetic stock, and advanced/improved cultivar.

As used herein, the term "clade" means a monophyletic group, defined as a group consisting of a single common ancestor and all its descendants.

The term "pathogen" as used herein is defined as any biological agent that causes disease or illness to its host. This includes bacteria, fungi, viruses and prions.

As used herein, "gene" means a hereditary unit (often indicated by a sequence of DNA) that occupies a specific location on a chromosome and that contains the genetic instruction for a particular phenotypic characteristics or trait in an organism such as a plant.

The term "nucleic acid" refers to deoxyribonucleotides or ribonucleotides and polymers thereof in either single-or double-stranded form, composed of monomers (nucleotides) containing a sugar, phosphate and a base which is either a purine or pyrimidine. Unless specifically limited, the term encompasses nucleic acids containing known analogs of natural nucleotides which have similar binding properties as the reference nucleic acid and are metabolized in a manner similar to naturally occurring nucleotides. Unless otherwise indicated, a particular nucleic acid sequence also implicitly encompasses conservatively modified variants thereof (e. g., degenerate codon substitutions) and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer *et al.,* 1991; Ohtsuka *et al.,* 1985; Rossolini *et al.* 1994). A "nucleic acid fragment" is a fraction of a given nucleic acid molecule. In higher plants, deoxyribonucleic acid (DNA) is the genetic material while ribonucleic acid (RNA) is involved in the transfer of information contained within DNA into proteins. The term "nucleotide sequence" refers to a polymer of DNA or RNA which can be single-or double-stranded, optionally containing synthetic, non-natural or altered nucleotide bases capable of incorporation into DNA or RNA polymers. The terms "nucleic acid" or "nucleic acid sequence" may also be used interchangeably with gene, cDNA, DNA and RNA encoded by a gene

The term "homology" is defined as (having) one or more similarities between characteristics that is due to their shared ancestry. In genetics, homology can be observed in DNA sequences that code for proteins (genes) and in non-coding DNA. For protein coding genes, one can compare translated amino acid sequences of different genes. For proteins, one can compare amino acid sequences of different proteins. Sequence homology may also indicate common function.

The terms "homolog" and "homologue" as used herein mean any gene or protein that is related to a second gene or protein by descent from a common ancestral DNA sequence. The term, homolog, may apply to the relationship between genes or proteins separated by the event of speciation (orthology) or to the relationship between genes or proteins separated by the event of genetic duplication (paralogy).

The term "homologous to" in the context of nucleotide or amino acid sequence identity refers to the similarity between the nucleotide sequences of two nucleic acid molecules or between the amino acid sequences of two protein molecules. Estimates of such homology are provided by either DNA-DNA or DNA-RNA hybridization under conditions of stringency as is well understood by those skilled in the art (as described in Haines and Higgins (eds.), Nucleic Acid Hybridization, IRL Press, Oxford, U. K.), or by the comparison of sequence similarity between two nucleic acids or proteins. Two nucleotide or amino acid sequences are homologous when their sequences have a sequence similarity of more than 50%, preferably more than 60%, 70%, 80%, 85%, 90%, 95%, or even 98%.

As used herein, the terms "ortholog" and "orthologue" are defined as any gene or protein in a species that is similar to one or more genes or proteins in other species. This includes orthologs arisen from a common ancestor. Genes or proteins that are found within one clade are orthologs, descended from a common ancestor. Orthologs often, but not always, have the same function. It also includes homologies that have arisen by convergent evolution.

The terms "genetic engineering", "recombinant DNA technology", "genetic modification/manipulation (GM)" and "gene splicing" as used herein all refer to techniques for direct manipulation of an organism's genes. These techniques are different from traditional breeding, where the organism's genes are manipulated indirectly. They use the techniques of molecular cloning and transformation to alter the structure and characteristics of genes directly. Genetic engineering techniques have found some successes in numerous applications, such as in improving crop technology, the manufacture of synthetic human insulin through the use of modified bacteria, the manufacture of erythropoietin in hamster ovary cells, and the production of new types of experimental mice such as the oncomouse (cancer mouse) for research. In general, genetic engineering, recombinant DNA technology, genetic modification/manipulation (GM) and comprise five main steps: 1) isolation of the genes of interest, 2) insertion of the genes into a transfer vector, 3) transfer of the vector to the organism to be modified, 4) transformation of the cells of the organism and 5) selection of the genetically modified organism (GMO) from those that have not been successfully modified.

The term "transformation" refers to the transfer of a nucleic acid fragment into the genome of a host cell, resulting in genetically stable inheritance. Host cells containing the transformed nucleic acid fragments are referred to as "transgenic" cells, and organisms comprising transgenic cells are referred to as "transgenic organisms". Examples of methods of transformation of plants and plant cells include *Agrobacterium*-mediated transformation (De Blaere *et al.,* 1987) particle bombardment technology (Klein *et al.* 1987; U.S. Patent No. 4,945,050), microinjection, CaPO₄ precipitation, lipofection (liposome fusion), use of a gene gun and DNA vector transporter (Kao *et al.,* 2008). Whole plants may be regenerated from transgenic cells by methods well known to the skilled artisan (see, for example, Fromm *et al.,* 1990).

The terms "transformed", "transgenic" and "recombinant" as used herein refer to a host organism such as a bacterium or a plant into which a heterologous nucleic acid molecule has been introduced. The heterologous nucleic acid molecule can be stably integrated into the genome generally known in the art and are disclosed in Sambrook *et al.,* 1989. See also Innis and Gelfand, 1999. For example, "transformed", "transformant", and "transgenic" plants or calli have been through the transformation process and contain a foreign gene integrated into their chromosome. The term "untransformed" refers to normal plants that have not been through the transformation process.

As used herein, "vector" is defined as any DNA molecule used as a vehicle to transfer foreign genetic material into another cell. This includes plasmids, bacteriophages and other viruses, cosmids, and artificial chromosomes. The vector may comprise an origin of replication, a multicloning site, and / or a selectable marker.

The term "expression vector" as used herein is defined as any expression construct that is used to introduce a specific nucleic acid into a target cell and express the protein that is encoded by that nucleic acid. Once the expression vector is inside the cell, the protein that is encoded by the nucleic acid may be produced by the cellular-transcription and translation machinery. This includes expression vectors that comprise regulatory sequences that act as enhancer and promoter regions and lead to efficient transcription of the nucleic acid carried on the expression vector. It also includes expression vectors that comprise a termination codon, adjustment of the distance between the promoter and the cloned gene, a transcription termination sequence and / or a PTIS (portable translation initiation sequence).

As used herein, "plasmid" means any extra-chromosomal DNA molecule separate from the chromosomal DNA which is capable of replicating independently of the chromosomal DNA. This includes any double-stranded generally circular DNA sequence that is capable of automatically replicating in a host cell.

As used herein, the term "virus" means any microscopic infectious agent that can only reproduce inside a host cell. This includes all non-enveloped viruses, i.e. viruses comprising a protein coat that protects the viruses' genes. It also includes all enveloped viruses, i.e. viruses further comprising an envelope of fat surrounding the viruses when they are outside the host cell. It includes all DNA viruses, RNA viruses and reverse transcription viruses.

The term "adenovirus" as used herein is defined as any non-enveloped icosahedral virus that is composed of a nucleocapsid and a double-stranded linear DNA genome and that belongs to the family *Adenoviridae.*

As used herein, the term "retrovirus" means any RNA virus that is replicated in a host cell via the enzyme reverse transcriptase to produce DNA from its RNA genome and that belongs to the family *Retroviridae.*

The term "virus-based" as used herein is defined as being based on any genetically-engineered virus carrying modified viral DNA or RNA that has been rendered noninfectious, but still comprises viral promoters and the nucleic acid to be translated, thus allowing for translation of the nucleic acid through a viral promoter.

A "marker gene" encodes a selectable or screenable trait. The term "selectable marker" refers to a polynucleotide sequence encoding a metabolic trait which allows for the separation of transgenic and non-transgenic organisms and mostly refers to the provision of antibiotic resistance. A selectable marker is for example the *aph*L1 encoded kanamycin resistance marker, the nptII gene, the gene coding for hygromycin resistance. Other selection markers are for instance reporter genes such as chloramphenicol acetyl transferase, β-galactosidase, luciferase and green fluorescence protein. Identification methods for the products of reporter genes include, but are not limited to, enzymatic assays and fluorimetric assays. Reporter genes and assays to detect their products are well known in the art and are described, for example in "Current Protocols in Molecular Biology", eds. Ausubel et al., Greene Publishing and Wiley-Interscience: New York (1987) and periodic updates.

The terms "peptide" and "protein" as used herein are used interchangeably and are defined as any polymer formed from amino acids that are linked in a defined order by amide bonds or peptide bonds. This includes all ribosomal peptides, non-ribosomal peptides, peptones, monopeptides, dipeptides, tripeptides, oligopeptides, polypeptides and peptide fragments.

The term "coding sequence" as used herein refers to a DNA or RNA sequence that codes for a specific amino acid sequence and excludes the non-coding sequences. It may constitute an "uninterrupted coding sequence", i.e., lacking an intron, such as in a cDNA or it may include one or more introns bound by appropriate splice junctions. An "intron" is a sequence of RNA which is contained in the primary transcript but which is removed through cleavage and re-ligation of the RNA within the cell to create the mature mRNA that can be translated into a protein.

As used herein, "regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences include enhancers, promoters, translation leader sequences, introns, and polyadenylation signal sequences. They include natural and synthetic sequences as well as sequences which may be a combination of synthetic and natural sequences. As is noted above, the term "suitable regulatory sequences" is not limited to promoters.

The term "promoter" as used herein refers to a nucleotide sequence, usually upstream (5') to its coding sequence, which controls the expression of the coding sequence by providing the recognition for RNA polymerase and other factors required for proper transcription. "Promoter" includes a minimal promoter that is a short DNA sequence comprised of a TATA box and other sequences that serve to specify the site of transcription initiation, to which regulatory elements are added for control of expression. "Promoter" also refers to a nucleotide sequence that includes a minimal promoter plus regulatory elements that is capable of controlling the expression of a coding sequence or functional RNA. This type of promoter sequence consists of proximal and more distal upstream elements, the latter elements often referred to as enhancers. Accordingly, an "enhancer" is a DNA sequence which can stimulate promoter activity and may be an innate element of the promoter or a heterologous element inserted to enhance the level or tissue specificity of a promoter. It is capable of operating in both orientations (normal or flipped), and is capable of functioning even when moved either upstream or downstream from the promoter. Both enhancers and other upstream promoter elements bind sequence-specific DNA-binding proteins that mediate their effects. Promoters may be derived in their entirety from a native gene, or be composed of different elements derived from different promoters found in nature, or even be comprised of synthetic DNA segments. A promoter may also contain DNA sequences that are involved in the binding of protein factors which control the effectiveness of transcription initiation in response to physiological or developmental conditions.

As used herein, the term "constitutive promoter" refers to a promoter that is able to express the open reading frame (ORF) that it controls in all or nearly all of the plant tissues during all or nearly all developmental stages of the plant.

The term "expression" as used herein refers to the transcription and/or translation of an endogenous gene, open reading frame (ORF) or portion thereof, or a transgene in plants. Expression refers to the transcription and stable accumulation of sense (mRNA) or functional RNA. Expression may also refer to the production of protein.

The term "constitutive expression" as used herein refers to expression using a constitutive promoter. "Transient expression" is expression as a result of a transient transformation event. Transient expression of a gene refers to the expression of a gene that is not integrated into the host chromosome but functions independently, either as part of an autonomously replicating plasmid or expression cassette, for example, or as part of another biological system such as a virus.

The term "primer" as used herein refers to an oligonucleotide which is capable of annealing to the amplification target allowing a DNA polymerase to attach thereby serving as a point of initiation of DNA synthesis when placed under conditions in which synthesis of primer extension product which is complementary to a nucleic acid strand is induced, i.e., in the presence of nucleotides and an agent for polymerization such as DNA polymerase and at a suitable temperature and pH. The (amplification) primer is preferably single stranded for maximum efficiency in amplification. Preferably, the primer is an oligodeoxyribonucleotide. The primer must be sufficiently long to prime the synthesis of extension products in the presence of the agent for polymerization. The exact lengths of the primers will depend on many factors, including temperature and source of primer. A "pair of bidirectional primers" as used herein refers to one forward and one reverse primer as commonly used in the art of DNA amplification such as in PCR amplification.

As used herein, the term "probe" means a single-stranded oligonucleotide sequence that will recognize and form a hydrogen-bonded duplex with a complementary sequence in a target nucleic acid sequence analyte or its cDNA derivative.

The terms "stringency" or "stringent hybridization conditions" refer to hybridization conditions that affect the stability of hybrids, e.g., temperature, salt concentration, pH, formamide concentration and the like. These conditions are empirically optimised to maximize specific binding and minimize non-specific binding of primer or probe to its target nucleic acid sequence. The terms as used include reference to conditions under which a probe or primer will hybridise to its target sequence, to a detectably greater degree than other sequences (e.g. at least 2-fold over background). Stringent conditions are sequence dependent and will be different in different circumstances. Longer sequences hybridise specifically at higher temperatures. Generally, stringent conditions are selected to be about 5°C lower than the thermal melting point (Tm) for the specific sequence at a defined ionic strength and pH. The Tm is the temperature (under defined ionic strength and pH) at which 50% of a complementary target sequence hybridises to a perfectly matched probe or primer.

Typically, stringent conditions will be those in which the salt concentration is less than about 1.0 M Na+ ion, typically about 0.01 to 1.0 M Na+ ion concentration (or other salts) at pH 7.0 to 8.3 and the temperature is at least about 30°C for short probes or primers (e.g. 10 to 50 nucleotides) and at least about 60°C for long probes or primers (e.g. greater than 50 nucleotides). Stringent conditions may also be achieved with the addition of destabilizing agents such as formamide. Exemplary low stringent conditions or "conditions of reduced stringency" include hybridization with a buffer solution of 30% formamide, 1 M NaCl, 1% SDS at 37°C and a wash in 2x SSC at 40°C. Exemplary high stringency conditions include hybridization in 50% formamide, 1 M NaCl, 1% SDS at 37°C, and a wash in 0.1x SSC at 60°C. Hybridization procedures are well known in the art and are described in e.g. Ausubel, F.M., Brent, R., Kingston, R.E., Moore, D.D.,Seidman, J.G., Smith, J.A., Struhl, K. eds. (1998) Current protocols in molecular biology. V.B. Chanda, series ed. New York: John Wiley & Sons.

### Description

Apart from Ecp6 so far homologues of the *C. fulvum* effector proteins have never been identified in other fungal species (Bolton *et al.* 2008). However, the inventors have found that several other fungi belonging to the Dothideomycetes clustering in the family of the *Mycosphaerellaceae,* including *M. fijiensis, C. nicotianae, C. beticola, C. zeina,* and *C. apii* produce effectors that are functionally related to those of *C. fulvum.* By (i) structural homology search using BLAST, (ii) mining of sequenced fungal genomes for homologous nucleotide sequences and protein motifs and (iii) using PCR-based techniques and chromosome walking, the inventors have now identified for the first time orthologs of the *C. fulvum* Avr4 effectors in the fungal pathogens *M. fijiensis, C. nicotianae, C. beticola, C. zeina, and C. apii* and have deduced their amino acid sequences as shown in Figure 3. This indicates that the *C. fulvum* Avr and Ecp effector homologues occur as orthologs in related plant pathogens that, however, infect and cause disease on totally unrelated host plant species like banana (monocot), sugar beet and tobacco (dicots).

In particular, orthologs with various degrees of identity to the Avr4 protein of *C. fulvum* were identified in *M. fijiensis* (34% iden.), *C. nicotianae* (32% iden.), *C. beticola* (27% iden.), *C. apii* (27% iden.), and *C. zeina,* (32% iden.), but it is expected that they do occur in all major groups of fungal species belonging to the order of the Dothideomycetes.

Eight cysteine residues are present in the *C. fulvum* Avr4 protein, which are involved in the formation of disulfide bridges occurring in proteins that bind chitin of the CBM14 superfamily (Chitin binding Peritrophin-A; see Figures 1 and 2). Avr4 protects fungal cell-wall chitin against plant chitinases in vitro and during infection. These cysteine residues are also conserved in the Avr4 orthologs of *M. fijiensis, C. nicotianae, C. beticola, C. zeina, and C. apii,* suggesting that those orthologs are also capable of binding chitin. Indeed, the inventors demonstrate that the *M. fijiensis* Avr4 ortholog produced in *Pichia pastoris* also binds chitin, indicating that it is a genuine ortholog or functional homologue of the *C. fulvum* Avr4 (Figure 7). The practical implication of this important finding is demonstrated in the Example, wherein the inventors demonstrate that co-expression of the *M. fijiensis* Avr4 ortholog and tomato Cf4 protein elicits a HR response in tobacco plants. Thus, the resistance of plants other than tomato plants to fungi belonging to the Dothideomycetes, other than *Cladosporium fulvum*, producing Avr4-orthologs can be increased by providing these plants with Cf4 protein.

The inventors therefore provide a method for increasing the resistance of a plant or part thereof that is susceptible to infection with a pathogen comprising an ortholog of the Avr4 protein of *Cladosporium fulvum,* wherein said method comprises transforming said plant or part thereof with a nucleic acid encoding for Cf4 or a functional homologue thereof and wherein said plant is not a tomato or tobacco plant. It will be clear to a skilled person that the method can be applied to any plant other than a tomato plant that is susceptible to infection with a pathogen comprising an ortholog of the Avr4 protein of *Cladosporium fulvum.* Preferably, said plant or part thereof is selected from the group consisting of a banana plant, a wheat plant, a sugar beet plant, a maize plant or a celery plant. For example, the resistance of a banana plant to *M. fijiensis* may be increased by transforming the banana plant with a nucleic acid encoding for the Cf4 protein.

Plants may be transformed with any nucleic acid encoding for a functional homologue of Cf4, i.e. a protein comprising an amino acid sequence that shows a high degree of similarity to the amino acid sequence of Cf4 (not being Cf4 itself) and that is capable to recognize Avr4 and orthologs thereof. As a skilled person will appreciate, homologous proteins can be found by applying any suitable screening method known in the art. For example, structural homology searches can be performed in databases, either at protein or DNA level. Also, sequenced plant genomes can be mined for homologous nucleotide sequences and protein motifs. When applying mining methods, amplification techniques such as PCR-based techniques may be applied to amplify the found DNA sequences.

Homologous proteins can be found in different plants. For example, the inventors found that functional Cf4 homologues termed Hcr9-Avr4s are present in species belonging to the *Lycopersicon* lexicon in the *Solanum* genus: *S. hirsutum* (Genbank accession number AJ002235), S. *chilense* (AY634610), *S. chmielewskii* (AY634611), *S. parviflorum* (AY634612) and *S. peruvianum* (AY634613). These proteins are 93-98% identical to each other, as can be observed in Table 1. Thus, said functional homologue is preferably an Hcr9-Avr4 peptide.

**Table 1: Amino acid alignment scores of the Hcr9-Avr4 homologues from S. hirsutum, S. peruvianum, S. chmielewskii, S. chilense and S. parviflorum.**

| SeqA | Name | Len(aa) | SeqB | Name | Len(aa) | Score |
|---|---|---|---|---|---|---|
| 1 | *S_parviflorum* | 807 | 2 | *S_hirsutum* | 806 | 93 |
| 1 | *S_parviflorum* | 807 | 3 | *S_peruvianum* | 807 | 98 |
| 1 | *S_parviflorum* | 807 | 4 | *S_chmielewskii* | 807 | 98 |
| 1 | *S_parviflorum* | 807 | 5 | *S_chilense* | 807 | 95 |
| 2 | *S*_*hirsutum* | 806 | 3 | *S_peruvianum* | 807 | 93 |
| 2 | *S*_*hirsutum* | 806 | 4 | *S_chmielewskii* | 807 | 93 |
| 2 | *S*_*hirsutum* | 806 | 5 | *S_chilense* | 807 | 97 |
| 3 | *S_peruvianum* | 807 | 4 | *S_chmielewskii* | 807 | 98 |
| 3 | *S_peruvianum* | 807 | 5 | *S_chilense* | 807 | 94 |
| 4 | *S_chmielewskii* | 807 | 5 | *S_chilense* | 807 | 94 |

Methods for transforming an organism with a foreign nucleic acid are known in the art, as described in for example "Gene Transfer: Delivery And Expression of DNA And RNA, A Laboratory Manual", Friedmann and Rossi ed., 2006 Cold Spring Harbor Laboratory Press, Cold Spring harbor, USA. For example, it is well known that an organism can be transformed with a nucleic acid by introducing a vector comprising the nucleic acid.

Virtually any nucleic acid delivery vehicle may be used for delivery to recipient plant cells. For example, DNA segments in the form of plasmids, *Agrobacterium* binary vectors and viral vectors such as virus-based expression vectors, or linear DNA fragments, in some instances containing only the DNA element to be expressed in the plant, and the like, may be employed. When employing virus-based vectors, such vectors are preferably based on an adenovirus or a retrovirus. The construction of vectors which may be employed in conjunction with the present invention will be known to those of skill of the art in light of the present disclosure (see, e. g., Sambrook *et al.,* 1989; Gelvin *et al.,* 1990). Vectors, including but not limited to plasmids, cosmids, YACs (yeast artificial chromosomes), BACs (bacterial artificial chromosomes) and DNA segments for use in transforming cells, according to the present invention will, of course, comprise the cDNA, gene or genes necessary for production of the desired protein in the transformant.

The vector of the invention can be introduced into any plant. The genes and sequences to be introduced can be conveniently used in expression cassettes for introduction and expression in any plant of interest. The transcriptional cassette will include in the 5'-to-3' direction of transcription, transcriptional and translational initiation regions, a DNA sequence of interest, and transcriptional and translational termination regions functional in plants.

The termination region may be native with the transcriptional initiation region, may be native with the DNA sequence of interest, or may be derived from another source.

Convenient termination regions are available from the Ti-plasmid of *A*. *tumefaciens,* such as the octopine synthase and nopaline synthase termination regions. See also, Guerineau et al. (1991) Mol. Gen. Genet. 262: 141-144; Proudfoot (1991) Cell 64: 671- 674; Sanfacon et al. (1991) Genes Dev. 5: 141-149; Mogen et al. (1990) Plant Cell 2: 1261-1272; Munroe et al. (1990) Gene 91: 151-158; Ballas et al. (1989) Nucleic Acids Res. 17: 7891-7903; Joshi et al. (1987) Nucleic Acid Res. 15: 9627-9639.

Methodologies for the construction of plant transformation constructs are described in the art.

Obtaining sufficient levels of transgene expression in the transformed plant is an important aspect in the production of genetically engineered crops. Expression of heterologous DNA sequences in a plant host is dependent upon the presence of an operably linked promoter that is functional within the plant host.

Transformation of plants can be undertaken with a single DNA molecule or multiple DNA molecules (i. e., co-transformation), and both these techniques are suitable for use with the expression cassettes of the present invention. Numerous transformation vectors are available for plant transformation, and the expression cassettes of this invention can be used in conjunction with any such vectors. The selection of vector will depend upon the preferred transformation technique and the target species for transformation.

Suitable methods of transforming plant cells include, but are not limited to, microinjection (Crossway *et al.,* 1986), electroporation (Riggs *et al.,* 1986), *Agrobacterium*-mediated transformation (Hinchee *et al.,* 1988), direct gene transfer (Paszkowski *et al.,* 1984), and ballistic particle acceleration using devices available from Agracetus, Inc., Madison, Wis. And BioRad, Hercules, Calif. (see, for example, Sanford et al., U. S. Pat. No. 4,945,050; and McCabe *et al.,* 1988). Also see Weissinger *et al.,* 1988; Sanford *et al.,* 1987 (onion); Christou *et al.,* 1988 (soybean); McCabe *et al.,* 1988 (soybean); Datta *et al.,* 1990 (rice); Klein *et al.,* 1989 (maize); Fromm *et al.,* 1990 (maize); and Gordon- Kamm *et al.,* 1990 (maize); Svab *et al.,* 1990 (tobacco chloroplast); Koziel *et al.,* 1993 (maize); Shimamoto *et al.,* 1989 (rice); Christou *et al.,* 1991 (rice); European Patent Application EP 0 332 581 (orchard grass and other *Pooideae*); Vasil *et al.,* 1992 (wheat); Weeks *et al.,* 1993 (wheat). For example, the resistance of a banana plant to *M. fijiensis* is increased by transforming the banana plant with an expression vector comprising a nucleic acid encoding for an Hcr9-Avr4 protein.

Other transformation methods are available to those skilled in the art, such as direct uptake of foreign DNA constructs (see EP 0295959), techniques of electroporation (Fromm *et al.,* 1986) or high velocity ballistic bombardment with metal particles coated with the nucleic acid constructs (Klein *et al.,* 1987, and U. S. Patent No. 4,945,050). Once transformed, the cells can be regenerated by those skilled in the art.

Those skilled in the art will appreciate that the choice of method might depend on the type of plant, i.e., whether a plant is monocotyledonous such as a banana or dicotyledonous such as sugar beet and tobacco plants.

In order to provide a quick and simple test if a new plant species indeed can yield a hypersensitive response upon presentation of the effector proteins of the invention, the person skilled in the art can perform one of two tests. One of the most reliable is the infiltration of the elicitor protein in the leaves, and scoring for the HR. Another one is a rapid transient expression test known under the name of ATTA (*Agrobacterium tumefaciens* Transient expression Assay). In this assay (of which a detailed description can be found in Van den Ackerveken, G., et al., Cell 87, 1307-1316, 1996) the nucleotide sequence coding for an effector protein is placed under control of the CaMV 35S promoter and introduced into an *Agrobacterium* strain which is also used in protocols for stable transformation. After incubation of the bacteria with acetosyringon or any other phenolic compound which is known to enhance *Agrobacterium* T-DNA transfer, 1 ml of the *Agrobacterium* culture is infiltrated into an *in situ* plant by injection after which the plants are placed in a greenhouse. After 2-5 days the leaves can be scored for occurrence of HR symptoms.

Also provided is a method for screening the resistance of a plant or a part thereof to at least one pathogen with the proviso that said pathogen is not *Cladosporium fulvum,* said method comprising: a) screening said at least one pathogen for presence of the Avr4 protein of *Cladosporium fulvum* or an ortholog thereof, b) selecting at least one pathogen comprising said Avr4 protein of *Cladosporium fulvum* or an ortholog thereof, c) introducing said at least one pathogen to said plant or part thereof, and d) screening said plant or part thereof for the absence or presence of a defense response to said at least one pathogen, wherein said plant is not a tomato plant. Preferably, said plant or part thereof is selected from the group consisting of a banana plant, a wheat plant, a maize plant, a celery plant or a sugar beet plant. Preferably, said ortholog comprises a chitin-binding domain similar to that found in members of the CBM14 superfamily of chitin-binding proteins. More preferably, said ortholog comprises an amino acid sequence that is selected from the group consisting of SEQ ID NO: 1, SEQ ID NO: 2, SEQ ID NO: 3, SEQ ID NO: 4, SEQ ID NO: 5 and SEQ ID NO: 6., more preferably selected from the group consisting of SEQ ID NO: 2, SEQ ID NO: 3, SEQ ID NO: 4, SEQ ID NO: 5 and SEQ ID NO: 6. Also preferred is screening a tobacco plant with an ortholog of Avr4.

Methods for screening pathogens for peptides are known in the art. A pathogen may be screened for the presence of Avr4 or an ortholog thereof by applying any method or technique deemed suitable by a person skilled in the art. Such methods include, but are not limited to, protein electrophoresis such as SDS-PAGE, electroblotting, immunoblot or Western blot, protein immunostaining, protein immunoprecipitation, protein assay, mass spectrometry such as MALDI-TOF and ESI-TOF, and chromatography such as HPLC and RPC. For example, several pathogens are screened for the presence of Avr4 or an ortholog thereof by Western blot. Based on the outcome of the Western blot, a pathogen comprising Avr4 is selected and introduced to a banana plant. Also screening methods based on bioinformatics may be applied. Also, mathematical similarity searches, such as for example BLAST, are very suitable for screening peptides in pathogens for similarity to Avr4.

One method to introduce the pathogen comprising the Avr4 protein or an ortholog thereof to a plant that is not a tomato plant is by directly applying the pathogen onto a part of the plant, e.g. a leaf or a stem segment. Alternatively, the pathogen may be injected. In a plant that does not have the functional cognate resistance gene the application of the pathogen will cause disease. However, in a plant that harbours a functional resistance gene, the Cf4 protein or functional homologue thereof will recognize the Avr4 or ortholog effector protein that is expressed by the pathogen and the cascade leading to the hypersensitive reaction will be started. As discussed before, and as is shown in Figure 4, this will lead to a local necrosis of the plant tissue, or, in a less strong reaction, to chlorosis of the injected site, which effect can be visually observed.

In stead of directly applying the pathogen onto a plant or part thereof it is also possible to introduce the pathogen indirectly to the plant by transforming the plant with a nucleic acid encoding for the Avr4 protein or ortholog thereof. One of the most suitable ways to enable expression of the protein in a plant or plant part is through the so-called ATTA (*Agrobacterium tumefaciens* Transient expression Assay), in which an *Agrobacterium tumefaciens* bacterium is provided with a construct encoding an effector protein according to the invention. Since the nucleotide sequences encoding the effector proteins are disclosed in this description or can be derived from the amino acid sequences that are also provided in the present description, any person of skill will be able to clone a coding sequence into an expression vector that is suitable for *Agrobacterium* transformation.

Alternatively, a viral vector can be used. A nucleic acid encoding the Avr4 effector protein or an ortholog thereof can be cloned into a viral vector after which the viral particles are used to infect the plant or plant part and to express the protein. Using a viral vector in which the virus stills maintains its infectious properties has a further advantage. Next to the observation of local necrosis or chlorosis, which indicates the presence of the resistance gene, the absence of the resistance gene will also be visible, because in such plants the disease that is caused by the virus will develop. Thus, even if no clear signs of a hypersensitive response (HR) will be detected, a lack of disease progress will indicate the presence of a resistance mechanism. Of course, such a system will only be feasible with plants that are normally susceptible for the virus that is used as a viral vector.

Ideally, it should be ensured that the Avr4 effector protein or ortholog thereof is excreted to the apoplastic space, because this is the location where the effector protein in nature is found. When the screening method is based on injecting the protein into the plant, the protein automatically will be present in the apoplastic space. If a screening method is applied wherein Avr4 or an ortholog thereof is introduced to a plant by a nucleic acid delivery vehicle having the nucleic acid encoding Avr4 or an ortholog thereof, expression into the apoplastic space can be achieved by providing the nucleotide sequence with a signal sequence for extracellular targeting. Such signal sequences are available for a person skilled in the art and one example is the signal sequence of the tobacco PR-1a gene. Alternative sequences can be obtained from the Avr4 peptide, the carrot extension gene or from studies on N-terminal signal sequences (Small, I. et al., 2004, Proteomics 4:1581-1590).

It is envisaged that both the method for increasing plant resistance and the method for screening plant resistance as described above will be of use when breeding or constructing plants with a fungal resistance based on the presence of the Avr4/ortholog-Cf4/homologue resistance mechanism. Thus, further provided is the use of a method for increasing the resistance of a plant or part thereof to at least one pathogen, wherein said plant is not a tomato or tobacco plant. Even further provided is the use of a method for screening the resistance of a plant or a part thereof to at least one pathogen, said pathogen not being *C. fulvum,* wherein said plant is not a tomato plant.

In case of a breeding program for the introduction of Avr4/ortholog-Cf4/homologue based resistance, the breeder normally will depart from a plant that contains Cf4 or a functional homologue thereof. This plant line will then be used as a parent line and crossed with another plant to generate offspring. This offspring can be tested on the presence of the resistance mechanism by applying the screening method as described above. This process then will be repeated until the desired end product is obtained. In stead of breeding, it will also be possible to engineer the resistance using recombinant techniques. In that case the gene encoding the resistance gene will be cloned into an appropriate vector and a target plant will be transformed with this vector. Functional expression of the resistance gene can then be assessed by applying a screening method wherein the plant is provided with the Avr4 effector protein or an ortholog thereof.

Next to utilizing existing traits of resistance from *Solanum* or *Nicotiana* species in molecular resistance breeding programs in order to achieve broad spectrum resistance, in monocot and dicot hosts species attacked by Dothideomycete fungal pathogens except *C. fulvum,* that produce functional homologues of *C. fulvum* Avr4 effector, it will be clear that the invention can also be extended to the identification of new resistance traits in banana, sugar beet, tobacco, maize, and celery by virus-based expression vectors expressing Avr4 homologues of the above mentioned fungi.

Several promoters have been isolated that can direct strong near-constitutive expression in monocot and/or dicot plants, including the maize ubiquitin promoter (Christensen *et al.* 1996), the rice actin1 promoter (McElroy *et al.* 1990), various enhanced cauliflower mosaic virus (CaMV) 35S promoters (Mitsuhara *et al.* 1996, Kay *et al.* 1987, Vai *et al.* 1996), the sugarcane bacilliform badnavirus (ScBV) (Schenk *et al.* 1999), the synthetic pEmu promoter (Last *et al.* 1991), the banana bunchy top virus (BBTV) (Hermann *et al.* 2001), the cassava vein mosaic virus (CVMV) (Verdaguer *et al.* 1996), and others (Schünmann *et al.* 2003) that can be used in these methods as well.

The invention is exemplified by the following example. This example is intended to illustrate the invention, without limiting the scope thereof in any way.

### EXAMPLE

### Materials and Methods

DNA manipulations were carried out by standard protocols (Sambrook *et al.* 1989). PCRs were performed with Super*Taq* (HT Biotechnology Ltd) or *Pfu* (Stratagene), according to the manufacturer's instructions. Restriction enzymes, T4 ligase, and *Escherichia coli* DH5a cells were from Life Technologies (Breda, The Netherlands). Primers were synthesized by Sigma-Aldrich. Tomato and *Nicotiana benthamiana* plants were grown under standard greenhouse conditions. For PVX inoculations four-week-old Money Maker (MM)-Cf0 (no known Cf resistance genes) and MM-Cf4 (carrying the *Cf-4* resistance gene) tomato lines were used, while for agroinfiltrations six to eight-week-old *N. benthamiana* plants were used. Authenticity of all generated constructs was confirmed by sequencing at Macrogen Inc.

### Heterologous production of His₆-FLAG-tagged MfAvr4 in Pichia pastoris

Plasmids for the expression of an N-terminal His₆-FLAG-tagged MfAvr4 protein in the methylotrophic yeast *P. pastoris* were generated as described before (Rooney *et al.* 2005). In brief, vector pPIC-9 (Invitrogen) was modified by inserting a cassette, containing a DNA fragment encoding for the His₆-tag, while the *Sma*I, *Apa*I, and SacII restriction sites were introduced for cloning purposes, resulting in vector pPIC-9His. To create His₆-FLAG-tagged MfAvr4, the ORF of *MfAvr4* encoding for the mature MfAvr4 protein without the signal peptide sequence and after intron removal was amplified from cDNA of the fungus using *Pfu* polymerase (Stratagene) and primers Avr4F_pichia_FLAGtagged (gactacaaggacgacgatgacaagGCAACGATGCAG GTGCGAAC; primer is phosphorylated at its 5' end) and Avr4R_pchia+EcoRIsite (TCAGAATTCTTACAGTTGTCGCATCC). Subsequently, the amplified product was cloned into pPIC-9His with the *Sma*I and *Eco*RI restriction sites. The plasmid was then introduced into *P. pastoris* strain GS115 (Invitrogen) by electroporation and transformants were grown on YPD plates. All the media used for growth of *P. pastoris,* i.e. MD, YPD, and BMMY, were made following the manufacturers' instructions (Invitrogen). To verify that the *MfAvr4* was successfully cloned in frame into the AOX1 locus and that there were no PCR-introduced mistakes in the coding sequence, automated DNA sequencing (Macrogen Inc.) was performed on the re-isolated plasmids from cultures of the obtained *P. pastoris* transformants using the AOXR (GCAAATGGCATTCTGACATCC) and AOXF (GACTGGTTCCAATTGACAAGC) priming sites present in pPIC-9. High cell density fermentation of *P. pastoris* transformants for large-scale MfAvr4 production was performed according to the procedure described by Stratton *et al.* (1998). In brief, starter cultures of 50 mL were grown for 2 days at 30°C (OD600 > 10), and were used to inoculate a 2 L vessel containing 900 mL FM22 medium. Starter cultures originated from a fresh colony grown on a MD or YPD plate. After autoclaving, the pH was adjusted to 4.9 with 5.0 M KOH or 25% (w/v) NH₄OH. Agitation was kept at 1200 rpm, and the airflow was maintained at 1-2 vvm to keep the dissolved oxygen (DO) levels at least above 30%. If needed, excessive foaming was prevented by adding a few droplets of Antifoam 289 (Sigma). Approximately 20 h after inoculation, glycerol depletion was observed by a sharp increase of the DO. At this stage the glycerol-feed was started at a rate of 10 mL ·L-1 ·h-1 (glycerol fed-batch phase), and properly adjusted to maintain a steady DO reading (near 35%). After 4 h, the methanol feed was started at a rate of 3.4mL ·L-1 ·h-1. The methanol feed rate was stepwise increased to 6 mL ·L-1 ·h-1 as soon as the culture had fully adapted to growth on methanol (4-6 h). To prevent methanol accumulation a DO spike was performed (a sharp increase in the DO levels has to occur after a halt of the methanol supply; Stratton et al., 1998).

### Chitin-binding assays

We examined whether MfAvr4 has specific affinity for chitin and no insoluble polysaccharides other than chitin, using an affinity precipitation assay (Van den Burg *et al.* 2006). In parallel, we included Avr4 from *C. fulvum* in these experiments as a positive control (data not shown). His₆-FLAG-tagged MfAvr4 protein was obtained from crude culture filtrates of *P. pastoris* transformants after fermentation and binding was examined as previously described (Van den Burg *et al.* 2006). Briefly, MfAvr4 (100 µL of *P. pastoris* culture filtrate) was incubated with ~10 mg/mL insoluble polysaccharides (crab shell chitin, chitin-agarose resin, chitosan, cellulose, and xylan; all Sigma, except for the resin: New England Biolabs) in 1.8 mL binding buffer consisting of 50 mM Tris.HCl pH8, 150 mM NaCl, and 1 x protease inhibitor. The polysaccharide fractions had been pre-equilibrated in the binding buffer. After two hrs of gentle rocking (15 rpm) at ambient temperature, the insoluble fraction was pelleted by centrifugation (3 min, 13,000×g) and the supernatant was collected. The protein fraction in the recovered supernatant was precipitated adding 10% final concentration (w/v) TCA (trichloroacetic acid) and incubated for 1hr on ice. After centrifugation (16,000×g for 30 min), the protein pellet was washed in - 20°C 80% acetone (v/v) and re-dissolved in SDS-PAGE sample buffer (1% sodium dodecyl sulfate, 100 mM DTT, 100mM Tris.HCl pH6.8) by boiling. The insoluble polysaccharides were three times washed in binding buffer. The bound protein fraction was eluted from the insoluble polysaccharides by boiling for 10 min in SDS-PAGE sample buffer. Equal amounts of the supernatant protein fraction and the bound protein fraction were loaded and separated on standard 20% SDS-PAGE and MfAvr4 was detected by immunoblotting using an anti-Flag monoclonal antibody (M2, Sigma).

### Agroinfiltration assays.

Transient co-expression of *MfAvr4* with *Cf-4* or other genes of interest, through infiltration of *Agrobacterium* cultures into leaf tissue of the model plant *N. benthamiana* was made as follows. For *in planta* expression of *MfAvr4* cDNA, the sequence encoding the signal peptide for extracellular targeting of the encoded MfAvr4 was replaced by that of the extracellular protein PR-1a of tobacco (Hammond-Kosack *et al.* 1994; Hammond-Kosack *et al.* 1995; Honée *et al.* 1998; Joosten *et al.* 1997). First, PR-1a was amplified using *Pfu* polymerase (Stratagene) with primers PR1a-F (GGCCATGGGATTTGTTCTCTTTTCAC) and PR1a-R (ATTTTGGGCACGGCAAGAG). The ORF of *MfAvr4* encoding for the mature MfAvr4 protein without its original signal peptide sequence and after intron removal (*MfAvr4mature*) was amplified from cDNA of the fungus using *Pfu* polymerase (Stratagene). Primers used for *MfAvr4mature* amplification were PR1a_MfAvr4-F (*CTTGCCGTGCCCAAAATGCT*GGAACGATGCAGGTGCG), introducing at the 5' end of the amplified product 17bps that were complementary to the 3' end of the PR-1a sequence (in *Italics* and underlined) and MfAvr4-R (GG*CTCGAG*TTACACGTTGTCGCATCCTG), introducing at the 3' end of the amplified product an *Xho*I site (in *Italics* and underlined). The fused PR-1a:MfAvr4 mature product was generated by an overlap extension PCR using Super*Taq* polymerase (HT Biotechnology Ltd) and was subsequently cloned into the pGEM®-T Easy Vector (Promega), according to the manufacturer's instructions (construct pHB1). Authenticity of the fusion product was examined by custom DNA sequencing (Macrogen Inc.), using the standard pUC/M13 Forward and Reverse Sequencing Primer binding sites present in the pGEM®-T Easy Vector. The fused PR-1a:MfAvr4 mature product was then excised from pHB1 with an *Nco*I and *Xho*I digest and inserted between the 35S promoter and the PI-II terminator in a likewise digested pRH80 vector (An *et al.* 1989, Van der Hoorn *et al.* 2000) (construct pHB2). Finally, the 35S::PR-1a:MfAvr4mature::PI-II cassette was excised from pHB2 and subsequently transferred into the binary plasmid pMOG800 (Honée *et al.* 1998, Van der Hoorn *et al.* 2000), with the use of *Xba*I and *EcoR*I restriction sites (construct pMfAvr4). This final pMfAvr4 construct was transformed into *Agrobacterium tumefaciens* strain GV301 by electroporation using standard procedures (Hood *et al.* 1993). Agroinfiltration of *N. benthamiana* plants was performed as described previously (Van der Hoorn *et al.* 2000). Constructs of the tomato resistance genes *Cf-4* (pCf4) and *Cf-9* (pCf9), and the *C. fulvum* effectors *Avr4* (pAvr4) and *Avr9* (pAvr9) in the binary expression vector pMOG800, were constructed as described for pMfAvr4 and were already available in the lab (Van der Hoorn et al. 2000).

### PVX-mediated in planta expression of MfAvr4

pSfinx (Takken *et al.* 2000), a binary vector that facilitates the expression of pathogen cDNAs under the control of the potato virus X (PVX) coat protein promoter, was used as a backbone for all PVX expression constructs used in this study. From left to right border, the T-DNA of this vector consists of a CaMV 35S promoter-driven PVX sequence containing the replicase gene, the triple gene block, the duplicated coat protein promoter, and the coat protein genes. The multiple cloning site of pSfinx for directed insertion of pathogen cDNAs (e.g. *Avr* genes) is located directly downstream of the duplicated coat protein promoter. pSfinx facilitates transfer of the expression construct to the plant cells by *A. tumefaciens,* while subsequent virus replication results in expression of the pathogen cDNA that can trigger an HR if the cDNA encodes an avirulence protein that is recognized by a cognate R gene in the plant. Plasmid pMfAvr4 described above, was used to amplify the fusion product PR-1a:MfAvr4mature with primers MfAvr4-F (GGCCCGGGGCTGGACGATGCAGGTGCG) and MfAvr4-R (GGCTCGAGTTACACGTTGTCGCATCCTG) that introduce an *Xma*I at 5' end and a *Xho*I site at the 3' end of the PR-1a:MfAvr4mature amplified product, respectively. PCRs were performed using Super*Taq* polymerase (HT Biotechnology Ltd) and the product was subsequently cloned into the pGEM®-T Easy Vector (Promega), according to the manufacturer's instructions (construct pHB4). The PR-1a:MfAvr4mature cassette was then excised from pHB4 with *Xma*I and *Xho*I and ligated into a likewise digested pSfinx vector, resulting in the final construct PVX::PR1a*MfAvr4.* The authenticity of the final construct was examined by custom DNA sequencing at Macrogen Inc. using primers OX10 (5'-CAATCACAGTGTTGGCTTGC-3') and N31 (5'-GACCCTATGGGCTGTGTTG-3') that flank the cDNA insert in the PVX backbone. The final PVX::PR1a*MfAvr4* construct was then transformed into *A*. *tumefaciens* strain GV301 by electroporation using standard procedures (Hood et al. 1993). Finally, transformants were cultured on plates containing modified LB medium (10 g 1_1 bacto-peptone; 5 g 1_1 yeast extract; 2.5 g 1_1 NaCl; 10 g 1_1 mannitol) for 48 h at 28°C and, subsequently, colonies were selected and inoculated on four-week-old tomato MM-Cf0 and MM-Cf4 tomato plants by toothpick inoculation (Hammond-Kosack et al. 1995). Systemic HR symptoms were recorded 10-14 days after inoculation (Fig. 6). As controls, PVX containing the empty pSfinx vector (PVX::pSfinx) and the *C. fulvum Avr4* in a binary vector (PVX::PR1a*Aur4*) were used. The later construct was already available in our lab (Joosten et al. 1997).

### REFERENCES

An G, Mitra A, Choi HK, Costa M.A, An K, Thornburg RW, Ryan CM. 1989. Functional analysis of the 3 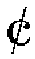 control region of the potato wound-inducible proteinase inhibitor II gene. *Plant Cell* 1:115-122.
Arzanlou M, Abeln ECA, Kema GHJ, Waalwijk C, Carlier J, de Vries I, Guzman M, Crous PW. 2007. Molecular diagnostics for the Sigatoka disease complex of banana. Phytopathology 97: 1112-1118.
Batzer MA, Carlton JE Deininger PL 1991. Enhanced evolutionary PCR using oligonucleotides with inosine at the 3'-terminus. Nucleic Acids Research 19: 5081.
De Blaere, R., Reynaerts, A., Hofte, H., Hernalsteens, J.-P., Leemans, J. and Van Montagu, M. 1987. Vectors for cloning in plant cells Methods in Enzymology 153, 277-291.
Bolton MD, Van Esse HP, Vossen JH, De Jonge R, Stergiopoulos I, Stulemeijer IJE, Van Den Berg GCM, Borras-Hidalgo O, Dekker HL, De Koster CG, De Wit PJGM, Joosten MHAJ, Thomma BPHJ. 2008. The novel Cladosporium fulvum lysin motif effector Ecp6 is a virulence factor with orthologues in other fungal species. Molecular Microbiology 69:119-36
Carlier J, Foure E, Gauhl F, Jones DR, Lepoivre P, Mourichon X, Pasberg-Gauhl C Romero RA. 2000. Black Leaf Streak. Pages 37-79 in: Diseases of Banana, Abaca and Enset. D. R. Jones.
Christensen AH, Quail PH. 1996. Ubiquitin promoter-based vectors for high-level expression of selectable and/or screenable marker genes in monocotyledonous plants. Transgenic Research 5: 213-218.
Christou P, McCabe DE, Swain WF. 1988 Stable Transformation of Soybean Callus by DNA-Coated Gold Particles, Plant Physiol. 87: 671-674.
Christou P, Ford TL, Kofron M. 1991. Production of transgenic rice (Oryza sativa L.) plants from agronomically important indica and japonica varieties via electric discharge particle acceleration of exogenous DNA into immature zygotic embryos. Trends in Biotechnology 10: 239-246.
Crossway, A, Oakes JV, Irvine JM, Ward B, Knauf VC, Shewmaker, LK 1986. Integration of foreign DNA following microinjection of tobacco mesophyll protoplasts. Molec. Gen. Genet. 202: 179-185.
Crouch JH, Vuylsteke D, Ortiz R. 1998. Perspectives on the application of biotechnology to assist the genetic enhancement of plantain and banana (Musa spp.). Electronic Journal of Biotechnology 1:1-12.
Datta, SK, Datta, K, Potrykus, I 1990. Embryogenesis and plant regeneration from microspores of both Indica and Japonica rice (Oryza sativa). Plant Sci. 67: 83-88.
De Kock MJD, Brandwagt BF, Bonnema G, De Wit PJGM, Lindhout P. 2005. The tomato Orion locus comprises a unique class of Hcr9 genes. Molecular Breeding 15:409-22.
De Kock MJD, Iskander HM, Brandwagt BF, Lauge R, De Wit PJGM, Lindhout P. 2004. Recognition of Cladosporium fulvum Ecp2 elicitor by non-host Nicotiana spp. is mediated by a single dominant gene that is not homologous to known Cf-genes. Molecular Plant Pathology 5: 397-408.
De Wit PJGM, Joosten MHAJ, Thomma BHPJ, Stergiopoulos I. 2008. Gene-for-gene models and beyond: the Cladosporium fulvum-tomato pathosystem. The MycotaV; Plant relationships, 2nd Edition, H.B. Deising (Ed). Springer-Verlag Berlin Heildelberg 2009. pp.135-156.
Dixon MS, Jones DA, Keddie JS, Thomas CM, Harrison K, Jones JDG. 1996. The tomato Cf-2 disease resistance locus comprises two functional genes encoding leucine-rich repeat proteins. Cell 84:451-59.
Fromm, ME, Taylor, LP, Walbot, V 1986 Stable transformation of maize after gene transfer by electroporation. Nature 319, 791-793.
Fromm, ME, Morrish, F, Armstrong, C, Williams, R, Thomas, J and Klein, TM. 1990. Inheritance and expression of chimeric genes in the progeny of transgenic maize plants. Bio/Technology 8: 833-839.
Gelvin SB, Habeck LL.1990. Vir genes influence the conjugal transfer of the Ti-plasmid of Agrobacterium tumefaciens. Journal of Bacteriology 172:1600-1608.
Gordon-Kamm WJ, Spencer TM, Mangano ML, Adams TR, Daines RJ, Start WG, O'Brien JV, Chambers SA, Adams Jr WR, Willetts NG, Rice TB, Mackey CJ, Krueger RW, Kausch AP, Lemaux PG. 1990. Transformation of maize cells and regeneration of fertile transgenic plants. The Plant Cell 2:603-618
Haanstra JPW, Lauge R, Meijer-Dekens F, Bonnema G, de Wit PJGM, Lindhout P. 1999. The Cf-ECP2 gene is linked to, but not part of, the Cf-4/Cf-9 cluster on the short arm of chromosome 1 in tomato. Molecular and General Genetics 262: 839-845.
Hammond-Kosack KE, Harrison K, Jones JDG. 1994. Developmentally regulated cell death on expression of the fungal avirulence gene Avr9 in tomato seedlings carrying the disease-resistance gene Cf-9. Proceedings of the National Academy of Sciences of USA 91:10445-10449
Hammond-Kosack KE, Staskawicz BJ, Jones JDG, Baulcombe DC. 1995. Functional expression of a fungal avirulence gene from a modified potato virus X genome. Molecular Plant-Microbe Interactions. 8:181-185.
Hermann SR, Becker DK, Harding RM Dale JL. 2001 Promoters derived from banana bunchy top virus-associated components S1 and S2 drive transgene expression in both tobacco and banana. Plant Cell Reports 20:642-646.
Hinchee MAW, Conner-Ward DV, Newell CA, McDonnell RE, Sato SJ, Gasser CS, Fischhoff DA, Re DB, Fraley RT, Horsch RB. 1988, Production of transgenic soybean plants using Agrobacterium-mediated DNA transfer. Bio/Technology 6:915-922.
Honée G, Buitink J, Jabs T, De Klo, J, Sijbolts F, Apotheker M, Weide R, Sijen T, Stuiver M, De Wit PJGM. 1998. Induction of defence-related responses in Cf9 tomato cells by the AVR9 elicitor peptide of Cladosporium fulvum is developmentally regulated. Plant Physiology 117:809-820.
Hood EE, Gelvin SB, Melchers LS, Hoekema A. 1993. New Agrobacterium helper plasmids for gene transfer to plants. Transgenic Resources 2:208-218.
Innis MA, Gelfand DH 1999: PCR Applications: protocols for functional genomics. Academic Press, San Diego, CA, USA.
Jones DR. 1993. Evaluating banana and plantain for reaction to black leaf streak disease in the south-pacific. Tropical Agriculture 70: 39-44
Jones DR. 2000. Diseases of banana, abaca and enset. CABI International, Wallingford, Oxon, UK.
Joosten MHAJ, Cozijnsen TJ, De Wit PJGM. 1994. Host resistance to a fungal tomato pathogen lost by a single base-pair change in an avirulence gene. Nature 367:384-86.
Joosten MHAJ, De Wit PJGM. 1999. The tomato-Cladosporium fulvum interaction: A versatile experimental system to study plant-pathogen interactions. Annual Review of Phytopathology 37:335-67.
Joosten MHAJ, Vogelsang R, Cozijnsen TJ, Verberne MC, De Wit PJGM. 1997. The biotrophic fungus Cladosporium fulvum circumvents Cf-4-mediated resistance by producing unstable AVR4 elicitors. Plant Cell 9:367-79.
Kao CY, Huang SH, Lin CM. 2008. A low-pressure gene gun for genetic transformation of maize (Zea mays L.). Plant Biotechnology Reports 2: 267-270.
Kay R, Chan A, Daly M, McPherson J. 1987. Duplication of CaMV 35S promoter sequences creates a strong enhancer for plant genes. Science 236: 1299-1302.
Klein TM, Wolf WD, Wu R, Sanford JC 1987: High velocity micro-projectiles for delivering nucleic acids into living cells. Nature 327:70-73.
Kooman-Gersmann M, Vogelsang R, Vossen P, van den Hooven HW, Mahe E, Honée G, De Wit PJGM. 1998. Correlation between binding affinity and necrosis-inducing activity of mutant Avr9 peptide elicitors. Plant Physiol. 117:609-18.
Klein, TM, Kornstein, L, Sanford, JC Fromm, ME 1989. Genetic transformation of maize cells by particle bombardment. Plant Physiol. 91: 440-444.
Koziel, MG, Beland GL, Bowman C, Carozzi NB, Crenshaw R, Crossland L, Dawson J, Desai N, Hill M, Kadwell S, Launis K, Lewis K, Maddox D, McPherson K, Meghji MR, Merlin E, Rhodes R, Warren GW, Wright M, Evola SV. 1993. Field performance of elite transgenic maize plants expressing an insecticidal protein derived from Bacillus thuringiensis. Bio/Technology 11:194-200.
Kruger J, Thomas CM, Golstein C, Dixon MS, Smoker M, Tang S, Mulder L, Jones JDG. 2002. A tomato cysteine protease required for Cf-2-dependent disease resistance and suppression of autonecrosis. Science 296:744-47.
Kruijt M, Kip DJ, Joosten MHAJ, Brandwagt BF, De Wit PJGM. 2005. The Cf-4 and Cf-9 resistance genes against Cladosporium fulvum are conserved in wild tomato species Molecular Plant-Microbe Interactions 18: 1011-1021.
Last DI, Brettell RIS, Chamberlaine DA, Chaudhury AM, Larkin PJ, Marsh EL, Peacock WJ, Dennis ES. 1991. pEmu: An improved promoter for gene expression in cereal cells. Theoretical Applied Genenetics 81: 581-588.
Laugé R, Goodwin PH, De Wit PJGM, Joosten MHAJ. 2000. Specific HR-associated recognition of secreted proteins from Cladosporium fulvum occurs in both host and non-host plants. Plant Journal 23:735-45.
Luderer R, Rivas S, Nurnberger T, Mattei B, Van den Hooven HW, Van der Hoorn RAL, Romeis T, Wehrfritz JM, Blume B, Nennstiel D, Zuidema D, Vervoort J, De Lorenzo G, Jones JDG, De Wit PJGM, Joosten MHAJ. 2001. No evidence for binding between resistance gene product Cf-9 of tomato and avirulence gene product AVR9 of Cladosporium fulvum. Molecular Plant-Microbe Interactions 14:867-76.
Luderer R, Takken FLW, De Wit PJGM, Joosten MHAJ. 2002. Cladosporium fulvum overcomes Cf-2-mediated resistance by producing truncated Avr2 elicitor proteins. Molecular Microbiology 45:875-84.
Marín D, Ching LD, Romero RA. 1992. Efecto de la Sigatoka negra sobre la productividad del plátano. Pages 85-86 in: Corporacion Bananera Nacional-Informe Anual 1991, San José, Costa Rica.
McCabe, DE, Swain WF, Martinell BJ, Christou P 1988,. Stable Transformation of Soybean (Glycine Max) by Particle Acceleration. Bio/Technology 6:923
McElroy D, Zhang W, Cao J, Wu R. 1990. Isolation of an efficient actin promoter for use in rice transformation. Plant Cell 2: 163-171.
Mitsuhara I, Ugaki M, Hirochika H, Ohshima M, Murakami T, Gotoh Y, Katayose Y, Nakamura S, Honkura R, Nishimiya S, Ueno K, Mochizuki A, Tanimoto H, Tsugawa H, Otsuki Y, Ohashi Y. 1996. Efficient promoter cassettes for enhanced expression of foreign genes in dicotyledonous and monocotyledonous plants. Plant Cell Physiology 37: 49-59.
Miya A, Albert P, Shinya T, Desaki Y, Ichimura K, Shirasu K, Narusaka Y, Kawakami N, Kaku H and Shibuya N. 2007. CERK1, a LysM receptor kinase, is essential for chitin elicitor signaling in Arabidopsis. Proceedings of the National Academy of Sciences USA 104: 19613-19618.
Ohtsuka E, Matsuki S, Ikehara M, Takahashi Y, Matsubara K. 1985, An alternative approach to deoxyoligonucleotides as hybridization probes by insertion of deoxyinosine at ambiguous codon positions. J Biol Chem. 260: 2605-2608.
Paszkowski, J, Shillito, RD, Saul, M, Mandak, V, Hohn, T, Hohn, B Potrykus, I 1984. Direct gene transfer to plants, EMBO J. 3: 2717-2722.
Riggs, CD Bates GW, 1986. Stable transformation of tobacco by electroporation: Evidence for plasmid concatenation. Proc. Natl. Acad. Sci. USA 83: 5602-5606.
Rooney HCE, van't Klooster JW, van der Hoorn RAL, Joosten MHAJ, Jones JDG, De Wit PJGM. 2005. Cladosporium Avr2 inhibits tomato Rcr3 protease required for Cf-2-dependent disease resistance. Science 308:1783-86.
Rossolini GM, Cresti S, Ingianni A, Catani P, Riccio ML, Satta G. 1994, Use of deoxyinosine-containing primers versus degenerate primers for polymerase chain reaction based on ambiguous sequence information. Molecular and Cellular Probes, 8: 91-98.
Sági L, Remy S, Swennen R 1998. Fungal disease control in banana, a tropical monocot: Transgenic plants in the third world? Phytoprotection (Suppl.) 79:117-120.
Sági L, Remy S, Swennen R. 1997. Genetic transformation for the improvement of bananas: A critical assessment. Focus paper No. 2. Pages 33-36 in: INIBAP Annual Report 1997. INIBAP, Montpellier, France.
Sambrook J, Fritsch EF, Maniatis TA. 1989. Molecular Cloning: A Laboratory Manual. 2nd ed. Cold Spring Harbor Laboratory, Cold Spring Harbor, NY.
Sanford, JC, Klein TM, Wolf ED, Allen N. 1987. Delivery of substances into cells and tissues using a microprojectile bombardment process. J. Particle Sci. Technol. 5:27-37.
Schenk PM, Sagi L, Remans T, Dietzgen RG, Bernard MJ, Graham MW, Manners JM. 1999. A promoter from sugarcane bacilliform badnavirus drives transgene expression in banana and other monocot and dicot plants. Plant Molecular Biology 39: 1221-1230.
Schünmann PHD, Surin B Waterhouse PM. 2003. A suite of novel promoters and terminators for plant biotechnology II. The pPLEX series for use in monocots. Functional Plant Biology 30: 453-460.
Shabab M, Shindo T, Gu C, Kaschani F, Pansuriya T, Chintha R, Harzen A, Colby T, Kamoun S, Van der Hoorn RA. 2008. Fungal effector protein AVR2 targets diversifying defense-related cys proteases of tomato. The Plant Cell 20:1169-83.
Shimamoto, K, Terada, R, Izawa, T, Fujimoto, H 1989. Fertile transgenic rice plants regenerated from transformed protoplasts. Nature 338: 274-276.
Soumpourou E, Iakovidis M, Chartrain L, Lyall V, Thomas CM. 2007. The Solanum pimpinellifolium Cf-ECP1 and Cf-ECP4 genes for resistance to Cladosporium fulvum are located at the Milky Way locus on the short arm of chromosome 1. Theoretical and Applied Genetics 115:1127-36.
Stergiopoulos I, De Kock MJD, Lindhout P, De Wit PJGM. 2007. Allelic variation in the effector genes of the tomato pathogen Cladosporium fulvum reveals different modes of adaptive evolution. Molecular Plant-Microbe Interactions 20:1271-83.
Stover RH 1980. Sigatoka leaf spot of bananas and plantains. Plant Disease 64:750-756.
Stover RH and Simmonds NW. 1987. Bananas. 3rd ed. Longman Scientific & Technical, Essex, England.
Stratton J, Chiruvolu V, Meagher M. 1998. High cell-density fermentation. In: Higgins DR, Cregg JM (eds) Methods in molecular biology: Pichia protocols. Humana, Totowa, pp 107-120
Svab Z, Hajdukiewicz P, Maliga P (1990): Stable transformation of plastids in higher plants. Proc Natl Acad Sci USA 87: 8526-8530.
Takken LW, Luderer R, Gabriëls SHEJ, Westerink N, Lu R. De Wit PJGM, Joosten MHAJ. 2000. A functional cloning strategy, based on a binary PVX-expression vector, to isolate HR-inducing cDNAs of plant pathogens. Plant Journal 24:275-283.
Thomas CM, Balint-Kurti PJ, Jones DA and Jones JDG. 1996. Plant pathogen resistance genes and uses thereof. International Patent Application Number: PCT/GB96/01155.
Thomma BPHJ, Van Esse HP, Crous PW, De Wit PJGM. 2005. Cladosporium fulvum (syn. Passalora fulva), a highly specialized plant pathogen as a model for functional studies on plant pathogenic Mycosphaerellaceae. Molecular Plant Pathology 6:379-93.
Vain P, Finer KR, Engler DE, Pratt RC, Finer JJ. 1996. Intronmediated enhancement of gene expression in maize (Zea mays L.) and bluegrass (Poa pratensis L.). Plant Cell Reports 15: 489-494.
Verdaguer B, de Kochko A, Beachy RN and Fauquet C. 1996. Isolation and expression in transgenic tobacco and rice plants of the cassava vein mosaic virus (CVMV) promoter. Plant Molecular Biology 31:1129-1139.
van den Ackerveken GFJM, van Kan JA, Joosten MHAJ, Muisers JM, Verbakel HM, De Wit PJGM. 1993. Characterization of two putative pathogenicity genes of the fungal tomato pathogen Cladosporium fulvum. Molecular Plant-Microbelinteractions 6:210-15.
van den Burg HA, Harrison SJ, Joosten MHAJ, Vervoort J, De Wit PJGM. 2006. Cladosporium fulvum Avr4 protects fungal cell walls against hydrolysis by plant chitinases accumulating during infection. Mol. Plant-Microbe Interactions 19:1420-30.
van den Burg HA, Spronk CAEM, Boeren S, Kennedy MA, Vissers JPC, Vuister GW, De Wit PJGM, Vervoort J. 2004. Binding of the AVR4 elicitor of Cladosporium fulvum to chitotriose units is facilitated by positive allosteric protein-protein interactions: The chitin-binding site of Avr4 represents a novel binding site on the folding scaffold shared between the invertebrate and the plant chitin-binding domain.. Journal of Biological Chemistry 279:16786-96.
van den Burg HA, Westerink N, Francoijs KJ, Roth R, Woestenenk E, Boeren S, De Wit PJGM, Joosten MHAJ, Vervoort J. 2003. Natural disulfide bond-disrupted mutants of AVR4 of the tomato pathogen Cladosporium fulvum are sensitive to proteolysis, circumvent Cf-4-mediated resistance, but retain their chitin binding ability. Journal of Biological Chemistry 278:27340-46.
van Esse HP, Bolton MD, Stergiopoulos I, De Wit PJGM, Thomma BPHJ. 2007. The chitin-binding Cladosporium fulvum effector protein Avr4 is a virulence factor. Molecular Plant-Microbe Interactions 20:1092-101.
van Esse HP, van 't Klooster JW, Bolton MD, Yadeta K, VanBaarlen P, Boeren S, Vervoort J, De Wit PJGM, Thomma BPHJ. 2008. The Cladosporium fulvum virulence protein Avr2 inhibits host proteases required for basal defense. Plant Cell 20: 1948-1963.
Van der Hoorn RAL, Laurent F, Roth R, De Wit PJGM. 2000. Agroinfiltration is a versatile tool that facilitates comparative analyses of Avr9/Cf-9-induced and Avr4/Cf-4-induced Necrosis. Molecular Plant-Microbe Interactions 13:439-446.
Van den Hooven HW, Van den Burg HA, Vossen P, Boeren S, De Wit PJGM and Vervoort J. 2001. Disulfide bond structure of the AVR9 elicitor of the fungal tomato pathogen Cladosporium fulvum: Evidence for a cystine knot. Biochemistry, 40: 3458-3466.
van Kan JAL, van den Ackerveken GFJM, De Wit PJGM. 1991. Cloning and characterization of cDNA of avirulence gene avr9 of the fungal pathogen Cladosporium fulvum, causal agent of tomato leaf mold. Molecular Plant-Microbe Interactions 4:52-59.
Vasil, V, Castillo AM, Fromm ME, Vasil IK.,. 1992. Herbicide resistant fertile transgenic wheat plants obtained by microprojectile bombardment of regenerable embryogenic callus. Bio/Technology 10:667-674.
Wan J, Xu Zhang XC, Neece D, Ramonell KM, Cloug S, Kim SY, Stacey MG, Stacey G. 2008. A LysM Receptor-Like Kinase Plays a Critical Role in Chitin Signaling and Fungal Resistance in Arabidopsis. The Plant Cell 20:471-481.
Weeks, JT, Anderson OD, Blechl AE. 1993. Rapid production of multiple independent lines of fertile transgenic wheat (Triticum aestivum). Plant Physiol. 102:1077-1084.
Weissinger, A, Tomes, D, Maddock, S, Fromm, M, Sanford, J. 1988, Maize transformation via micro-projectile bombardment, in Molecular Biology: Genetic Improvements of Agriculturally Important Crops: Progress and Issues Meeting, pp. 21-25, Cold Spring Harbor Laboratory, Cold Spring Harbor, New York.
Westerink N, Brandwagt BF, De Wit PJGM, Joosten MHAJ. 2004. Cladosporium fulvum circumvents the second functional resistance gene homologue at the Cf-4 locus (Hcr9-4E) by secretion of a stable avr4E isoform. Molecular Microbiology 54:533-45.

## Claims

1. A method for increasing the resistance of a plant or part thereof that is susceptible to infection with a pathogen comprising an ortholog of the Avr4 protein of *Cladosporium fulvum,* wherein said method comprises transforming said plant or part thereof with a nucleic acid encoding for Cf4 or a functional homologue thereof and wherein said plant is not a tomato or tobacco plant.

2. The method according to claim 1, wherein said plant or part thereof is selected from the group consisting of a banana plant, a wheat plant, a sugar beet plant, a maize plant and a celery plant.

3. The method according to claim 1 or 2, wherein said functional homologue is an Hcr9-Avr4 peptide.

4. The method according to any one of claims 1 to 3, wherein said transforming comprises introducing a nucleic acid delivery vehicle, preferably wherein said delivery vehicle is an expression vector.

5. The method according to claim 4, wherein said expression vector is a virus-based expression vector, preferably an adenovirus or a retrovirus.

6. A method for screening the resistance of a plant or a part thereof to at least one pathogen with the proviso that said pathogen is not *Cladosporium fulvum.*, said method comprising:
a) screening said at least one pathogen for presence of the Avr4 protein of *Cladosporium fulvum* or an ortholog thereof,
b) selecting at least one pathogen comprising said Avr4 protein of *Cladosporium fulvum* or an ortholog thereof,
c) introducing said at least one pathogen to said plant or part thereof, and
d) screening said plant or part thereof for the absence or presence of a defense response to said at least one pathogen,
wherein said plant is not a tomato or tobacco plant and preferably wherein said plant or part thereof is selected from the group consisting of a banana plant, a wheat plant, a sugar beet plant, a maize plant, and a celery plant.

7. The method according to claim 6, wherein said ortholog comprises a chitin-binding domain similar to that found in members of the CBM14 superfamily of chitin-binding proteins.

8. The method according to any one of claims 6 or 7, wherein said ortholog comprises an amino acid sequence that is selected from the group consisting of SEQ ID NO: 1, SEQ ID NO: 2, SEQ ID NO: 3, SEQ ID NO: 4, SEQ ID NO: 5 and SEQ ID NO: 6, preferably selected from the group consisting of SEQ ID NO: 2, SEQ ID NO: 3, SEQ ID NO: 4, SEQ ID NO: 5 and SEQ ID NO: 6.

9. The method according to any one of claims 1 to 8, wherein said pathogen is a fungus, preferably wherein said fungus is a Dothideomycete, more preferably wherein said Dothideomycete is selected from the group consisting of *Mycosphaerella fijiensis, Cercospora nicotianae, Cercospora beticola, Cercospora zeina,* and *Cercospora apii,* with the proviso that said pathogen is not *Cladosporium fulvum.*

10. Use of a method according to any one of claims 1 to 5 for increasing the resistance of a plant or part thereof to at least one pathogen, wherein said plant is not a tomato or tobacco plant.

11. Use of a method according to any one of claims 6 to 8 for screening the resistance of a plant or a part thereof to at least one pathogen, wherein said plant is not a tomato or tobacco plant.

12. The use according to claim 10 or 11, wherein said plant or part thereof is selected from the group consisting of a banana plant, a wheat plant, a sugar beet plant, a maize plant, or a celery plant.

13. The use according to any one of claims 10 to 12, wherein said pathogen is a fungus, preferably wherein said fungus is a Dothideomycete, more preferably wherein said Dothideomycete is selected from the group consisting of *Mycosphaerella fijiensis, Cercospora nicotianae, Cercospora beticola, Cercospora zeina,* and *Cercospora apii,* with the proviso that said pathogen is not *Cladosporium fulvum.*

14. Method for increasing the resistance of a plant or part thereof that is susceptible to infection with a pathogen, wherein said pathogen is a Dothideomycete, with the proviso that said pathogen is not Cladosporium fulvum, more preferably wherein said Dothideomycete is selected from the group consisting of Mycosphaerella fijiensis, Cercospora nicotianae, Cercospora beticola, Cercospora zeina, and Cercospora apii, wherein said method comprises transforming said plant or part thereof with a nucleic acid encoding for Cf4 or a functional homologue thereof, preferably wherein said plant is not a tomato or tobacco plant, more preferably wherein said plant is selected from the group consisting of a banana plant, a wheat plant, a sugar beet plant, a maize plant, and a celery plant.

## Patentansprüche

1. Verfahren zur Erhöhung der Resistenz einer Pflanze oder eines Teiles davon, die/der anfällig für Infektion mit einem Pathogen, umfassend ein Ortholog des Avr4 Proteins von *Cladosporium fulvum*, ist, wobei das Verfahren umfasst, dass die Pflanze oder der Teil davon mit einer Nukleinsäure, kodierend für Cf4 oder eines funktionellen Homologs davon, transformiert wird und wobei die Pflanze keine Tomaten- oder Tabakpflanze ist.

2. Verfahren nach Anspruch 1, wobei die Pflanze oder der Teil davon ausgewählt ist aus der Gruppe, bestehend aus einer Bananenpflanze, einer Weizenpflanze, einer Zuckerrübenpflanze, einer Maispflanze und einer Selleriepflanze.

3. Verfahren nach Anspruch 1 oder 2, wobei das funktionelle Homolog ein Hcr9-Avr4 Peptid ist.

4. Verfahren nach einem der Ansprüche 1 bis 3, wobei die Transformation umfasst, dass ein Nukleinsäureabgabevehikel eingeführt wird, bevorzugt wobei das Abgabevehikel ein Expressionsvektor ist.

5. Verfahren nach Anspruch 4, wobei der Expressionsvektor ein virusbasierter Expressionsvektor ist, bevorzugt ein Adenovirus oder ein Retrovirus.

6. Verfahren zum Screening der Resistenz einer Pflanze oder eines Teiles davon gegen mindestens ein Pathogen, mit der Maßgabe, dass das Pathogen nicht *Cladosporium fulvum* ist, wobei das Verfahren umfasst:
a) ein Screening des mindestens einen Pathogens auf die Anwesenheit des Avr4 Proteins von *Cladosporium fulvum* oder eines Orthologs davon,
b) Auswählen von mindestens einem Pathogen, umfassend das Avr4 Protein von *Cladosporium fulvum* oder eines Orthologs davon,
c) Einführen des mindestens einen Pathogens in die Pflanze oder des Teiles davon und
d) Screening der Pflanze oder des Teiles davon auf die Abwesenheit oder Anwesenheit einer Abwehrreaktion gegen das mindestens eine Pathogen,
wobei die Pflanze keine Tomaten- oder Tabakpflanze ist und bevorzugt wobei die Pflanze oder der Teil davon ausgewählt ist aus der Gruppe, bestehend aus einer Bananenpflanze, einer Weizenpflanze, einer Zuckerrübenpflanze, einer Maispflanze und einer Selleriepflanze.

7. Verfahren nach Anspruch 6, wobei das Ortholog eine Chitinbindende Domäne umfasst, ähnlich der, die in Mitgliedern der CBM14 Superfamilie Chitin-bindender Proteine vorzufinden ist.

8. Verfahren nach einem der Ansprüche 6 oder 7, wobei das Ortholog eine Aminosäuresequenz umfasst, die ausgewählt ist aus der Gruppe, bestehend aus SEQ ID NR: 1, SEQ ID NR: 2, SEQ ID NR: 3, SEQ ID NR: 4, SEQ ID NR: 5 und SEQ ID NR: 6, bevorzugt ausgewählt aus der Gruppe, bestehend aus SEQ ID NR: 2, SEQ ID NR: 3, SEQ ID NR: 4, SEQ ID NR: 5 und SEQ ID NR: 6.

9. Verfahren nach einem der Ansprüche 1 bis 8, wobei das Pathogen ein Pilz ist, bevorzugt wobei der Pilz ein Dothideomycetes ist, bevorzugter wobei der Dothideomycetes ausgewählt ist aus der Gruppe, bestehend aus *Mycosphaerella fijiensis, Cercospora nicotianae, Cercospora beticola, Cercospora zeina* und *Cercospora apii*, mit der Maßgabe, dass das Pathogen nicht *Cladosporium fulvum* ist.

10. Verwendung eines Verfahrens nach einem der Ansprüche 1 bis 5 zur Erhöhung der Resistenz einer Pflanze oder eines Teiles davon gegen mindestens ein Pathogen, wobei die Pflanze keine Tomaten- oder Tabakpflanze ist.

11. Verwendung eines Verfahrens nach einem der Ansprüche 6 bis 8 zum Screening der Resistenz einer Pflanze oder eines Teiles davon gegen mindestens ein Pathogen, wobei die Pflanze keine Tomaten- oder Tabakpflanze ist.

12. Verwendung nach Anspruch 10 oder 11, wobei die Pflanze oder ein Teil davon ausgewählt ist aus der Gruppe, bestehend aus einer Bananenpflanze, einer Weizenpflanzen, einer Zuckerrübenpflanze, einer Maispflanze oder einer Selleriepflanze.

13. Verwendung nach einem der Ansprüche 10 bis 12, wobei das Pathogen ein Pilz ist, bevorzugt wobei der Pilz ein Dothideomycetes ist, bevorzugter wobei der Dothideomycetes ausgewählt ist aus der Gruppe, bestehend aus *Mycosphaerella fijiensis, Cercospora nicotianae, Cercospora beticola*, *Cercospora zeina* und *Cercospora apii*, mit der Maßgabe, dass das Pathogen nicht *Cladosporium fulvum* ist.

14. Verfahren zur Erhöhung der Resistenz einer Pflanze oder eines Teiles davon, die/der anfällig für Infektion mit einem Pathogen ist, wobei das Pathogen ein Dothideomycetes ist, mit der Maßgabe, dass das Pathogen nicht *Cladosporium fulvum* ist, bevorzugter wobei der Dothideomycetes ausgewählt ist aus der Gruppe, bestehend aus *Mycosphaerella fijiensis*, *Cercospora nicotianae, Cercospora beticola*, *Cercospora zeina* und *Cercospora apii*, wobei das Verfahren umfasst, dass die Pflanze oder der Teil davon mit einer Nukleinsäure, kodierend für Cf4 oder einem funktionellen Homolog davon transformiert wird, bevorzugt wobei die Pflanze keine Tomaten- oder Tabakpflanze ist, bevorzugter wobei die Pflanze ausgewählt ist aus der Gruppe, bestehend aus einer Bananenpflanze, einer Weizenpflanze, einer Zuckerrübenpflanze, einer Maispflanze und einer Selleriepflanze.

## Revendications

1. Méthode pour augmenter la résistance d'une plante ou d'une partie de celle-ci qui est susceptible d'être infectée par un pathogène comprenant un orthologue de la protéine Avr4 du *Cladosporium fulvum*, où la méthode consiste à transformer ladite plante ou partie de celle-ci avec un acide nucléique codant pour Cf4 ou un homologue fonctionnel de celui-ci et dans laquelle la plante n'est pas un plant de tomate ni de tabac.

2. Méthode selon la revendication 1, dans laquelle ladite plante ou partie de celle-ci est sélectionnée parmi le groupe constitué d'un bananier, de blé, d'une betterave sucrière, d'un maïs et d'un céleri.

3. Méthode selon les revendications 1 ou 2, dans laquelle l'homologue fonctionnel est un peptide de Hcr9-Avr4.

4. Méthode selon l'une quelconque des revendications 1 à 3, dans laquelle la transformation consiste à introduire un véhicule d'apport en acide nucléique, de préférence dans laquelle le véhicule d'apport est un vecteur d'expression.

5. Méthode selon la revendication 4, dans laquelle le vecteur d'expression est un vecteur d'expression à base de virus, de préférence un adénovirus ou un rétrovirus.

6. Méthode pour cribler la résistance d'une plante ou d'une partie de celle-ci en ce qui concerne au moins un pathogène pourvu que ledit pathogène ne soit pas *Cladosporium fulvum,* ladite méthode consistant à :
a) cribler l'au moins un pathogène en ce qui concerne la présence de la protéine Avr4 de *Cladosporium fulvum* ou d'un orthologue de celle-ci,
b) sélectionner au moins un pathogène comprenant la protéine Avr4 de *Cladosporium fulvum* ou un orthologue de celle-ci,
c) introduire l'au moins un pathogène dans ladite plante ou partie de celle-ci, et
d) cribler ladite plante ou partie de celle-ci en ce qui concerne l'absence ou la présence d'une réponse de défense vis-à-vis dudit au moins un pathogène, dans laquelle ladite plante n'est pas un plant de tomate ni de tabac et de préférence dans laquelle ladite plante ou partie de celle-ci est sélectionnée parmi le groupe constitué d'un bananier, de blé, d'une betterave sucrière, d'un maïs, et d'un céleri.

7. Méthode selon la revendication 6, dans laquelle l'orthologue comprend un domaine de liaison à la chitine similaire à celui trouvé dans des éléments de la super famille CBM14 des protéines de liaison à la chitine.

8. Méthode selon l'une quelconque des revendications 6 ou 7, dans laquelle l'orthologue comprend une séquence d'acides aminés qui est sélectionnée parmi le groupe constitué de SEQ ID NO : 1, SEQ ID NO : 2, SEQ ID NO : 3 SEQ ID NO : 4, SEQ ID NO : 5 et SEQ ID NO : 6, de préférence sélectionnée parmi le groupe constitué de SEQ ID NO : 2, SEQ ID NO : 3 SEQ ID NO : 4, SEQ ID NO : 5 et SEQ ID NO : 6.

9. Méthode selon l'une quelconque des revendications 1 à 8, dans laquelle le pathogène est un champignon, de préférence dans laquelle le champignon est un Dothideomycete, de manière plus préférée dans laquelle le Dothideomycete est sélectionné parmi le groupe constitué de *Mycosphaerella fijiensis, Cercospora nicotianae, Cercospora beticola, Cercospora zeina*, et *Cercospora apii*, pourvu que le pathogène ne soit pas *Cladosporium fulvum.*

10. Utilisation d'une méthode selon l'une quelconque des revendications 1 à 5 pour augmenter la résistance d'une plante ou d'une partie de celle-ci en ce qui concerne au moins un pathogène, dans laquelle ladite plante n'est pas un plant de tomate ni de tabac.

11. Utilisation d'une méthode selon l'une quelconque des revendications 6 à 8 pour cribler la résistance d'une plante ou d'une partie de celle-ci en ce qui concerne au moins un pathogène, dans laquelle la plante n'est pas un plant de tomate ni de tabac.

12. Utilisation selon les revendications 10 ou 11, dans laquelle la plante ou partie de plante est sélectionnée parmi le groupe constitué d'un bananier, de blé, d'une betterave à sucre, d'un maïs et d'un céleri.

13. Utilisation selon l'une quelconque des revendications 10 à 12, dans laquelle le pathogène est un champignon, de préférence dans laquelle le champignon est un Dothideomycete, de manière plus préférée dans laquelle le Dothideomycete est sélectionné parmi le groupe constitué de *Mycosphaerella fijiensis, Cercospora nicotianae, Cercospora beticola, Cercospora zeina*, et *Cercospora apii*, pourvu que le pathogène ne soit pas *Cladosporium fulvum*.

14. Méthode pour augmenter la résistance d'une plante ou d'une partie de celle-ci qui est susceptible d'être infectée par un pathogène, dans laquelle le pathogène est un Dothideomycete, pourvu que le pathogène ne soit pas *Cladosporium fulvum,* de préférence dans laquelle le Dothideomycète est sélectionné parmi le groupe constitué de *Mycosphaerella fijiensis, Cercospora nicotianae, Cercospora beticola, Cercospora zeina*, et *Cercospora apii*, la méthode consistant à transformer la plante ou partie de celle-ci avec un acide nucléique codant pour Cf4 ou un homologue fonctionnel de celui-ci, de préférence dans laquelle la plante n'est pas un plant de tomate ni de tabac, de manière plus préférée dans laquelle la plante est sélectionnée parmi le groupe constitué d'un bananier, de blé, d'une betterave à sucre, d'un maïs et d'un céleri.


### SEQUENCE LISTING

<110> Wageningen Universiteit
   de Wit, Peter JGM
   Stergiopoulos, Ioannis
   Kema, Gerrit HJ
<120> Method for increasing the resistance of a plant or a part thereof to a pathogen, method for screening the resistance of a plant or part thereof to a pathogen, and use thereof.
<130> P88514PC00
<150> EP 09164957.4
   <151> 2009-07-08
<160> 18
<170> PatentIn version 3.3
<210> 1
   <211> 135
   <212> PRT
   <213> Cladosporium fulvum
<400> 1
   Met His Tyr Thr Thr Leu Leu Leu Ser Thr Leu Leu Val Gly Thr Ala
   1               5                   10                  15
   Leu Ala Gln Pro Thr Asn Pro Pro Ala Lys Thr Pro Lys Lys Ala Pro
               20                  25                  30
   Lys Thr Gln Pro Tyr Asn Pro Cys Lys Pro Gln Glu Val Ile Asp Thr
           35                  40                  45
   Lys Cys Met Gly Pro Lys Asp Cys Leu Tyr Pro Asn Pro Asp Ser Cys
       50                  55                  60
   Thr Thr Tyr Ile Gln Cys Val Pro Leu Asp Glu Val Gly Asn Ala Lys
   65                  70                  75                  80
   Pro Val Val Lys Pro Cys Pro Lys Gly Leu Gln Trp Asn Asp Asn Val
                   85                  90                  95
   Gly Lys Lys Trp Cys Asp Tyr Pro Asn Leu Ser Thr Cys Pro Val Lys
               100                 105                 110
   Thr Pro Gln Pro Lys Pro Lys Lys Gly Gly Val Gly Gly Lys Lys Ala
           115                 120                 125
   Ser Val Gly His Pro Gly Tyr
       130                 135
<210> 2
   <211> 121
   <212> PRT
   <213> Mycosphaerella fijiensis
<400> 2
   Met Leu Ser Thr Thr Arg Ile Thr Leu Leu Ala Thr Phe Ala Leu Ala
   1               5                   10                  15
   Thr Thr Ser Tyr Ala Gly Thr Met Gln Val Arg Asp Thr Ala Pro Ala
               20                  25                  30
   Phe Val Cys Pro Ser Ala Asp Ile Thr Ala Thr Lys Cys Leu Gly Pro
           35                  40                  45
   Lys Asp Cys Leu Tyr Pro Asn Pro Lys Thr Cys Asn Gly Tyr Ile Gln
       50                  55                  60
   Cys Ser Pro Ala Asp Ala Ser Tyr Thr Thr Gly Ile Val His Glu Met
   65                  70                  75                  80
   Pro Cys Pro Ser Gly Leu Gln Trp Asn Asp Asn Gln Lys Trp Cys Asp
                   85                  90                  95
   Trp Pro Glu Asn Ser Thr Cys Gly Asn Thr Gly Gly Thr Ile Tyr Arg
               100                 105                 110
   Cys Asp Glu Thr Gly Cys Asp Asn Val
           115                 120
<210> 3
   <211> 134
   <212> PRT
   <213> Cercospora nicotianae
<400> 3
   Met Tyr Gly Leu Phe His Leu Ala Val Leu Ser Ala Thr Ala Leu Ser
   1               5                   10                  15
   Ala Ser Ala Leu Pro Gln Gln Gln Glu Lys Pro Gly Val Asn Ala Asp
               20                  25                  30
   Phe Ile Cys Pro Pro Glu Asp Met Lys Arg Thr His Cys Met Gly Pro
           35                  40                  45
   Phe Asp Cys Leu Tyr Ala Leu Pro Gly Asn Cys Leu Gly Tyr Ile Gln
       50                  55                  60
   Cys Gln Pro Lys Asp Glu Thr Tyr Glu Thr Gly Ile Ala Tyr Glu Arg
   65                  70                  75                  80
   Pro Cys Arg Ala Gly Gln Trp Trp Asn Asp Glu Lys Lys Tyr Cys Asp
                       85                  90                  95
   Thr Val Glu Pro Pro Thr Cys Lys Ala Pro Val Val Pro Ala Ser Ala
               100                 105                 110
   Ala Pro Ala Pro Ala Pro Ala Pro Ala Pro Ala Ala Pro Ala Ala Pro
           115                 120                 125
   Ala Ser Thr Ser Pro Cys
       130
<210> 4
   <211> 135
   <212> PRT
   <213> Cercospora beticola
<400> 4
   Met Tyr Gly Leu Phe His Leu Ala Val Leu Ser Ala Thr Ala Pro Ser
   1               5                   10                  15
   Ala Ser Ala Leu Pro Gln Gln Gln Glu Lys Pro Gly Val Asn Lys Asp
               20                  25                  30
   Phe Ile Cys Pro Pro Glu Asp Met Lys Arg Thr His Cys Met Gly Pro
           35                  40                  45
   Phe Asp Cys Leu Tyr Ala Leu Pro Gly Asn Cys Leu Gly Tyr Ile Gln
       50                  55                  60
   Cys Gln Pro Lys Asp Thr Thr Tyr Glu Thr Gly Ile Ala Tyr Glu Arg
   65                  70                  75                  80
   Pro Cys Arg Ala Gly Gln Trp Trp Asn Asp Glu Lys Lys Tyr Cys Asp
                   85                  90                  95
   Thr Val Val Pro Pro Thr Cys Lys Ala Pro Val Val Pro Ala Ser Ala
               100                 105                 110
   Pro Ala Pro Ala Pro Ala Pro Ala Pro Ala Pro Ala Ala Pro Ala Ala
           115                 120                 125
   Pro Ala Ser Thr Ser Pro Cys
       130                 135
<210> 5
   <211> 134
   <212> PRT
   <213> Cercospora zeina
<400> 5
   Met Tyr Gly Leu Phe His Leu Ala Val Leu Ser Ala Thr Ala Leu Ser
   1               5                   10                  15
   Ala Ser Ala Leu Pro Gln Gln Gln Glu Lys Pro Gly Val Asn Ala Asn
               20                  25                  30
   Phe Ile Cys Pro Pro Glu Asp Met Lys Arg Thr His Cys Met Gly Pro
           35                  40                  45
   Phe Asp Cys Leu Tyr Ala Leu Pro Gly Asn Cys Leu Gly Tyr Ile Gln
       50                  55                  60
   Cys Gln Pro Lys Asp Glu Thr Tyr Glu Thr Gly Ile Ala Tyr Glu Arg
   65                  70                  75                  80
   Pro Cys Arg Ala Gly Gln Trp Trp Asn Asp Glu Lys Lys Tyr Cys Asp
                   85                  90                  95
   Thr Val Glu Pro Pro Thr Cys Lys Ala Pro Val Val Pro Ala Ser Ala
               100                 105                 110
   Ala Pro Ala Pro Ala Pro Ala Pro Ala Pro Ala Ala Pro Ala Ala Pro
           115                 120                 125
   Ala Ser Thr Ser Pro Cys
       130
<210> 6
   <211> 135
   <212> PRT
   <213> Cercospora apii
<400> 6
   Met Tyr Gly Leu Phe His Leu Ala Val Leu Ser Ala Thr Ala Pro Ser
   1               5                   10                  15
   Ala Ser Ala Leu Pro Gln Gln Gln Glu Lys Pro Gly Val Asn Lys Asp
               20                  25                  30
   Phe Ile Cys Pro Pro Glu Asp Met Lys Arg Thr His Cys Met Gly Pro
           35                  40                  45
   Phe Asp Cys Leu Tyr Ala Leu Pro Gly Asn Cys Leu Gly Tyr Ile Gln
       50                  55                  60
   Cys Gln Pro Lys Asp Thr Thr Tyr Glu Thr Gly Ile Ala Tyr Glu Arg
   65                  70                  75                  80
   Pro Cys Arg Ala Gly Gln Trp Trp Asn Asp Glu Lys Lys Tyr Cys Asp
                   85                  90                  95
   Thr Val Val Pro Pro Thr Cys Lys Ala Pro Val Val Pro Ala Ser Ala
               100                 105                 110
   Pro Ala Pro Ala Pro Ala Pro Ala Pro Ala Pro Ala Ala Pro Ala Ala
           115                 120                 125
   Pro Ala Ser Thr Ser Pro Cys
       130                 135
<210> 7
   <211> 44
   <212> DNA
   <213> Artificial
<220>
   <223> primer
<400> 7
   gactacaagg acgacgatga caaggcaacg atgcaggtgc gaac 44
<210> 8
   <211> 26
   <212> DNA
   <213> Artificial
<220>
   <223> primer
<400> 8
   tcagaattct tacagttgtc gcatcc 26
<210> 9
   <211> 21
   <212> DNA
   <213> Artificial
<220>
   <223> primer
<400> 9
   gcaaatggca ttctgacatc c 21
<210> 10
   <211> 21
   <212> DNA
   <213> Artificial
<220>
   <223> primer
<400> 10
   gactggttcc aattgacaag c 21
<210> 11
   <211> 26
   <212> DNA
   <213> Artificial
<220>
   <223> primer
<400> 11
   ggccatggga tttgttctct tttcac 26
<210> 12
   <211> 19
   <212> DNA
   <213> Artificial
<220>
   <223> primer
<400> 12
   attttgggca cggcaagag 19
<210> 13
   <211> 37
   <212> DNA
   <213> Artificial
<220>
   <223> primer
<400> 13
   cttgccgtgc ccaaaatgct ggaacgatgc aggtgcg 37
<210> 14
   <211> 28
   <212> DNA
   <213> Artificial
<220>
   <223> primer
<400> 14
   ggctcgagtt acacgttgtc gcatcctg 28
<210> 15
   <211> 27
   <212> DNA
   <213> Artificial
<220>
   <223> primer
<400> 15
   ggcccggggc tggacgatgc aggtgcg 27
<210> 16
   <211> 28
   <212> DNA
   <213> Artificial
<220>
   <223> primer
<400> 16
   ggctcgagtt acacgttgtc gcatcctg 28
<210> 17
   <211> 20
   <212> DNA
   <213> Artificial
<220>
   <223> primer
<400> 17
   caatcacagt gttggcttgc 20
<210> 18
   <211> 19
   <212> DNA
   <213> Artificial
<220>
   <223> primer
<400> 18
   gaccctatgg gctgtgttg 19